# EUROPEAN PATENT SPECIFICATION

(11) **EP 2 550 290 B1**
(45) Date of publication and mention of the grant of the patent: **02.12.2015**
(21) Application number: 11715594.5
(22) Date of filing: 23.03.2011
(51) Int. Cl.: C12P 21/02, C07K 1/06, C07K 1/107

(54) **Method of modifying a specific lysine**
Methode zur Modifikation eines spezifischen Lysins
Méthode de modifier une lysine spécifique

(30) Priority: 27.08.2010 GB 201014556; 24.03.2010 GB 201005046
(43) Date of publication of application: 30.01.2013
(73) Proprietor: Medical Research Council, Swindon SN2 1FL (GB)
(72) Inventor: CHIN, Jason, Cambridge CB2 0HQ (GB); VIRDEE, Satpal, Dundee DD1 SEH (GB)
(74) Representative: Richards, William John
(86) International application number: PCT/GB2011/000420
(87) International publication number: WO 2011/117583

(56) References cited:
- WO-A1-2009/056803
- WO-A2-2011/039518
- NGUYEN DUY P ET AL: "Genetically Encoding N-epsilon-Methyl-L-lysine in Recombinant Histones", JOURNAL OF THE AMERICAN CHEMICAL SOCIETY, AMERICAN CHEMICAL SOCIETY, WASHINGTON, DC; US, vol. 131, no. 40, 14 October 2009 (2009-10-14), pages 14194-14195, XP002618073, ISSN: 0002-7863, DOI: DOI:10.1021/JA906603S [retrieved on 2009-09-22]
- YANAGISAWA T ET AL: "Multistep Engineering of Pyrrolysyl-tRNA Synthetase to Genetically Encode N epsilon-(o-Azidobenzyloxycarbonyl) lysine for Site-Specific Protein Modification", CHEMISTRY AND BIOLOGY, CURRENT BIOLOGY, LONDON, GB, vol. 15, no. 11, 24 November 2008 (2008-11-24), pages 1187-1197, XP025678151, ISSN: 1074-5521, DOI: DOI:10.1016/J.CHEMBIOL.2008.10.004 [retrieved on 2008-11-21] cited in the application
- SATPAL VIRDEE ET AL: "Engineered diubiquitin synthesis reveals Lys29-isopeptide specificity of an OTU deubiquitinase", NATURE CHEMICAL BIOLOGY, vol. 6, no. 10, 1 October 2010 (2010-10-01), pages 750-757, XP055002769, ISSN: 1552-4450, DOI: 10.1038/nchembio.426
- GUOQIANG XU ET AL: "Global analysis of lysine ubiquitination by ubiquitin remnant immunoaffinity profiling", NATURE BIOTECHNOLOGY, vol. 28, no. 8, 1 August 2010 (2010-08-01) , pages 868-873, XP055002709, ISSN: 1087-0156, DOI: 10.1038/nbt.1654
- H. TRAN ET AL: "Trabid, a new positive regulator of Wnt-induced transcription with preference for binding and cleaving K63-linked ubiquitin chains", GENES & DEVELOPMENT, vol. 22, no. 4, 15 February 2008 (2008-02-15), pages 528-542, XP055002681, ISSN: 0890-9369, DOI: 10.1101/gad.463208

## Description

### Field Of The Invention

The invention relates to protein synthesis and modification. In particular the invention relates to lysine-linked protein modifications.

### Background To The Invention

Lysine residues are key determinants for post-translational protein modification and their use key post-translational modifications such as ubiquitination, methylation, and acetylation are well known. Selectively modifying specific lysine residues within a protein remains a problem.

Ubiquitination is a reversible post-translational modification in which a specific lysine residue in an acceptor protein forms an isopeptide bond with the C-terminus of the ubiquitin donor. While the role of ubiquitination in regulating protein stability via proteasomal targeting is well established, it is emerging that ubiquitin is involved in almost every aspect of biology, including cell signaling, intracellular trafficking and the response to DNA damage^{1,2}. Ubiquitin forms covalent chains through each of its Lysine residues (K6, K11, K27, K29, K33, K48, or K63), or through is N-terminus, and it is proposed that the distinct functions mediated by ubiquitin in diverse biological processes may be encoded in the distinct properties of the different ubiquitin chains ^{2,3}.

While proteomic studies reveal that all chains types are present *in vivo* ^{4,5}, most is known about K48 chains and K63 chains, which are important in proteasomal degradation and cell signaling, respectively ^{1,6}. In contrast very little is known about the other so-called 'atypical' linkages, though they account for more than half of the ubiquitin linkages found in model organisms, where proteomics data exists⁵. A central challenge in studying the roles of specific ubiquitin chains is to synthesize homogeneous chains bearing defined linkages.

D1 (WO2009/056803) discloses a tRNA synthetase capable of binding Ne-acetyl lysine. In particular, a tRNA synthetase capable of binding Ne-acetyl lysine having at least 90% sequence identity to the amino acid sequence of MbPyIRS is described.

D2 (NGUYEN el al 2009 JOURNAL OF THE AMERICAN CHEMICAL SOCIETY, vol. 131, no. 40, pages 14194-14195) discloses genetically encoding N-epsilon-methyl-L-lysine in recombinant histones.

D3 (YANAGISAWA et al 2008 CHEMISTRY AND BIOLOGY vol. 15, no. 11, pages 1187-1197) discloses multistep engineering of pyrrolysyl-tRNA synthetase to genetically encode N-epsilon-(o-azidobenzyloxycarbonyl) lysine for site-specific protein modification.

The present invention seeks to overcome problem(s) associated with the prior art.

### Summary Of The Invention

The present inventors have solved the problem of specifically targeting individual lysine residues for modification within a polypeptide chain. One aspect of this is to genetically encode the chemical protection for the target lysine(s). This enables site-specific protection of specific residue(s) within the polypeptide chain.

More importantly, the inventors have developed techniques for differentially protecting the lysines in the polypeptide. Specifically, the inventors teach use of a chemical protection which differs from the genetically encoded protection.

In this way, a polypeptide may be produced having a target lysine protected site-specifically by genetically encoding protection of that residue. This polypeptide with its site-specific protected lysine can then be chemically treated to protect each of the remaining (unprotected) lysine residues with a different chemical protection group. This dual approach has the advantage that the finished polypeptide has two different types of chemical protection on its lysines. This enables the original target lysine to be selectively deprotected by chemistry leaving the other protection groups on the other lysines intact. In this way the target lysine is site-specifically deprotected and can therefore be modified whilst the other lysines remain unaffected.

Finally all of the unmodified lysines can be optionally deprotected to leave nature-identical lysine residues if desired.

The present invention is based on this ingenious differential chemistry approach to the selective protection/deprotection of target lysine(s) in the polypeptide sequence.

Thus in one aspect the invention provides a method of modifying a specific lysine residue in a polypeptide comprising at least two lysine residues, said method comprising
(a) providing a polypeptide comprising a target lysine residue protected by a first protecting group, and at least one further lysine residue;
(b) treating the polypeptide to protect said further lysine residue(s), wherein the protecting group for said further lysine residues is different to the protecting group for the target lysine residue;
(c) selectively deprotecting the target lysine residue; and
(d) modifying the deprotected lysine residue of (c),
wherein providing the polypeptide comprises
(i) providing a nucleic acid encoding the polypeptide which nucleic acid comprises an orthogonal codon encoding the target lysine;
(ii) translating said nucleic acid in the presence of an orthogonal tRNA synthetase/tRNA pair capable of recognising said orthogonal codon and incorporating said target lysine residue protected by a first protecting group into the polypeptide chain.

Suitably said orthogonal codon comprises TAG, said tRNA comprises *Mb*tRNA_{CUA} and said tRNA synthetase comprises MbPyIRS.

Suitably the target lysine residue protected by a first protecting group is chosen from the group consisting of: Nε-(t-butyloxycarbonyl)-L-lysine.

Suitably the target lysine residue protected by a first.protecting group is Nε-(t-butyloxycarbonyl)-L-lysine.

Suitably the protecting group for said further lysine residues is chosen from the group consisting of: N-(benzyloxycarbonyloxy) succinimide (Cbz-Osu). When the protecting group for said further lysine residues is N-(benzyloxycarbonyloxy) succinimide (Cbz-Osu), suitably it is supplied in basic DMSO.

Suitably the protecting group for said further lysine residues is N-(benzyloxycarbonyloxy) succinimide (Cbz-Osu).

Suitably step (b) comprises treating the polypeptide with *N-*(benzyloxycarbonyloxy)succinimide (Cbz-OSu) in basic DMSO.

Suitably the amount of Cbz-OSu used is determined as [molar amount equivalent to the amount of polypeptide being treated] multiplied by [number of lysines to be protected in polypeptide plus one]. This advantageously provides a slight excess of the protecting groups for reaction with the lysines to be protected and therefore helps to propel the reaction towards completion (saturation/homogeneity).

Suitably step (c) comprises treating the polypeptide with trifluoroacetic (TFA) acid in water.

Suitably the modification of step (d) comprises
(i) activating thioester by conversion to *N*-hydroxysuccinimidyl ester in the presence of Ag(l);
(ii) adding a polypeptide to be joined to the target lysine; and
(iii) incubating to allow formation of a specific isopeptide bond.

Suitably the modification of step (d) is carried out on the ε-amino group of the target lysine residue.

Suitably multiple modifications may be made to the target lysine in step (d).

Suitably the method as described above further comprises the step: (e) deprotecting said further lysine residue(s). Suitably step (e) comprises treating the polypeptide with a mixture of trifluoromethanesulfonic acid (TFMSA):trifluoroacetic acid (TFA):dimethylsulfide (DMS) in the ratio 1:3:6.

One advantage of deprotecting all remaining lysines as after modification is complete is to restore the polypeptide as close as possible to its natural form.

Suitably step (a) comprises producing the polypeptide by genetically incorporating the target lysine residue protected by a first protecting group into the polypeptide chain during its translation.

Suitably the polypeptide is ubiquitin.

Suitably the modification of step (d) is the covalent linkage of a further polypeptide chain to the target lysine. Suitably the further polypeptide chain is ubiquitin. In this embodiment the invention may be advantageously applied to ubiquitination of polypeptide(s).

In some embodiments the polypeptide is ubiquitin and the further polypeptide chain is ubiquitin. In these embodiments the invention is advantageously applied to the manufacture of ubiquitin chains. These chains may be made in any of the K-linked forms of ubiquitin (e.g. K6, K11, K27, K29, K33, K48, or K63 linked ubiquitin) simply by selecting the appropriate lysine residue to target in the first polypeptide.

When multiple modifications are made, steps (c)-(d) may be repeated to produce a chain of polypeptides joined by covalent linkages through lysine residues. Clearly this may involved repeated deprotection at the end of each round of modification before moving on to the next reaction in the sequence of modifications. Alternatively no protection/deprotection may be needed for the subsequent modifications if the reaction chemistry used for them is already specific to the target lysine (or modified target lysine) from earlier round(s) of modification.

Also described are polypeptide(s) produced as described above. Said polypeptide(s) may comprise a K-linked ubiquitin chain. Said K-linkage may be a K6, K11, K27, K29, K33, K48, or K63 linkage. Suitably said K-linkage is a K6 or K29 linkage.

Also described is use of TRABID as a K29 deubiquitinase.

Also described is a method of cleaving K29 linked ubiquitin comprising contacting same with TRABID.

Also described is a ubiquitin polypeptide comprising at least one protected lysine residue. Ubiquitin polypeptide comprising one or more of K6Boc, K29Boc.

Also described is a nucleic acid encoding ubiquitin wherein at least one lysine codon is replaced with an orthogonal codon.

Also described is use of K-linked ubiquitins of the invention for example for use in activating or promoting a response to DNA damage, and/or for use in preventing or treating cancer such as early-onset breast or ovarian cancer. Such uses may advantageously be applied in medicine.

Preferred methods of the invention may also be referred to in the following specification as GOPAL (**G**enetically-encoded **O**rthogonal **P**rotection and **A**ctivated **L**igation).

Suitably the targeted reaction in the method according to the invention is used to link proteins together.

Also described are the proteins obtainable from said method. Suitably, the proteins are ubiquitins.

Also described is homogenously linked ubiquitin chains in which the ubiquitin polypeptides are linked by the lysine amino acid residue at position 6 and the C-terminal. Said ubiquitin chains are for use in activating or promoting a response to DNA damage and therefore for use in preventing or treating cancer.

### Description of the Drawings

The invention will now be described in relation to the drawings in which:

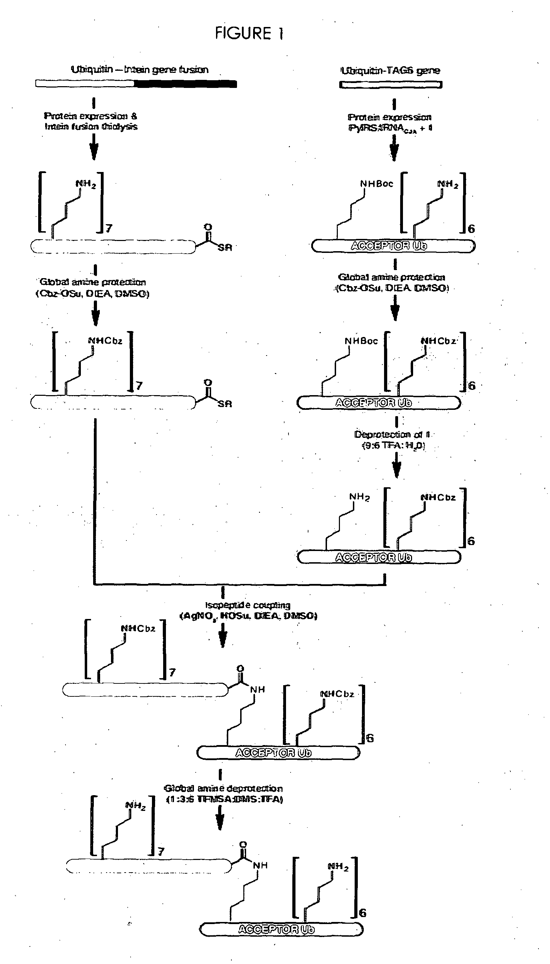

**Figure 1** **-** GOPAL strategy for site-specific isopeptide bond formation, exemplified for the synthesis of K6-linked diubiquitin. **1** is *N*ε-*(t-*butyloxycarbonyl)-L-lysine, shown in blue. Cbz-OSu is N-(benzyloxycarbonyl) succinimide which reacts with the *N*ε-amine of lysine in proteins to give *N*ε-(benzyloxycarbonyl)-L-lysine. TFA is trifluoroacetic acid, DIEA is N, N diisopropylethylamine, DMSO is dimethyl sulfoxide, HOSu is N-(hydroxy)succinimide, TFMSA is trifluoromethanesulfonic acid, DMS is dimethyl sulfide.

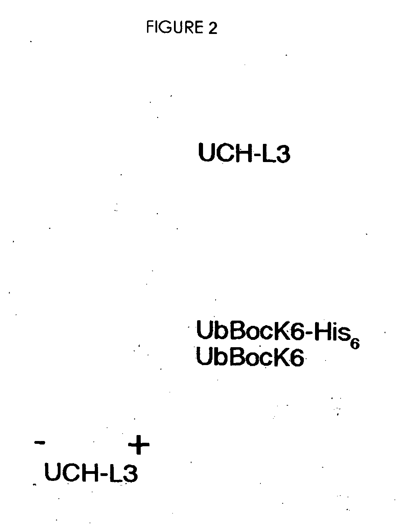

**Figure 2** **-** Cleavage of the hexahistidine tag from UbBocK6-His6 with UCH-L3.

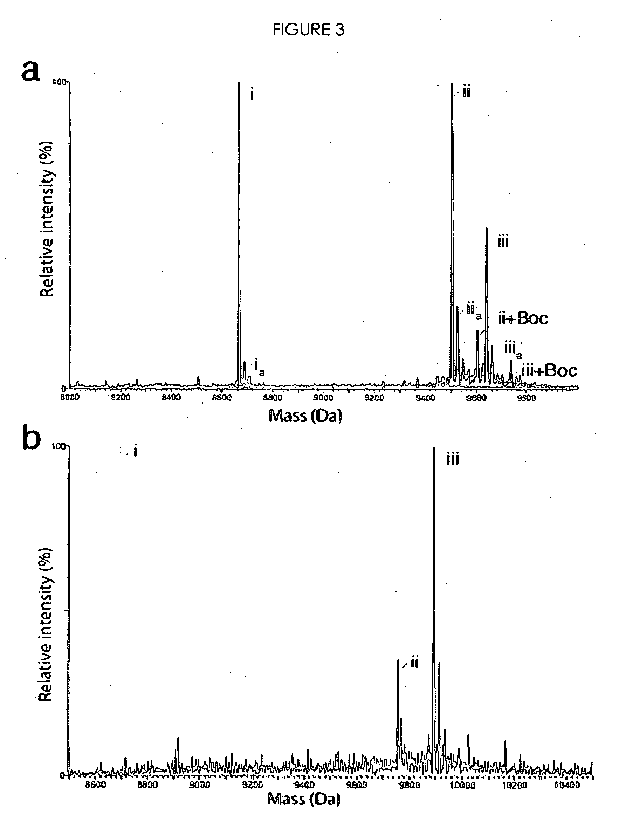

**Figure 3** **-** Characterization of the donor and acceptor ubiquitin reaction partners for isopeptide bond formation. **A.** Electrospray ionization mass spectrometry (ESI-MS) of the acceptor ubiquitin. Ubiquitin with **1** genetically incorporated at position 6 (UbBocK6, green trace, **i**). The C-terminal His tag in UbBocK6-his6 was removed with UCH-L3 (observed mass = 8665 Da; calculated = 8665 Da), **ia** is the Na⁺ adduct. The blue ESI-MS spectra shows UbBocK6 in which the free amines have been chemically protected with Cbz and the Boc protecting group of **1** at position 6 has been selectively deprotected. **ii** is UbK6(Cbz₇), observed mass = 9501.5 Da, calculated = 9503 Da; **iia** is UbK6(Cbz₇) + Na⁺; **iii** is UbK6(Cbz₈), observed mass = 9636.5 Da, calculated = 9637 Da; **iiia** is UbK6(Cbz₈) + Na⁺, **iia** and **iia** correspond to small amounts of UbBocK6(Cbz₇) and UbBoc6(Cbz₈) respectively, resulting from incomplete deprotection of the Boc group in **1** by TFA. **B.** Purified ubiquitin-MES thioester (UbSR, orange trace, **i**). Observed mass=8690 Da, calculated mass=8689 Da. The blue ESI-MS trace shows UbSR in which the free amines have been chemically protected with the Cbz protecting group. **ii** is UbSR(Cbz₈), observed mass = 9760 Da, calculated mass = 9761 Da; **iii** is UbSR(Cbz₉), observed mass = 9895 Da, calculated mass = 9895 Da.

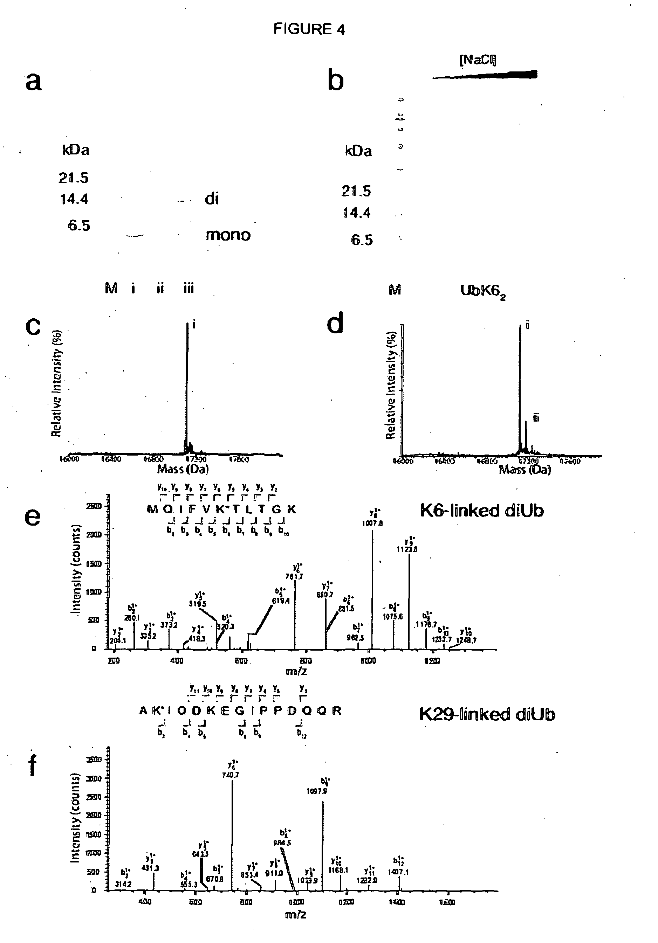

**Figure 4** **-** Synthesis, purification and characterization of K6- and K29-linked diubiquitin. **A.** SDS-PAGE analysis of isopeptide forming reaction between UbBocK6(Cbz₇₋₈) and UbSR(Cbz₇₋₈). **Lane M,** broad range molecular weight marker (Bio-Rad); **lane i,** concentration normalized UbSR(Cbz₇₋₈) input; **lane ii,** concentration normalized UbK6(Cbz₇₋₈) input; **lane iii,** The K6 isopeptide bond forming reaction mixture after 16 h. **B.** Eluted fractions of pure UbK6₂ after MonoS cation exchange chromatography. **C.** ESI-MS analysis of purified K6-linked diubiquitin (UbK6₂) demonstrates the formation of an isopeptide bond (observed mass = 17113, calculated = 17112). **D.** ESI-MS analysis of purified K29-linked diubiquitin (UbK29₂). **i** is the formation of an isopeptide bond (observed mass = 17111, calculated = 17112); **ii** is the nitrate salt (observed mass = 17174, calculated = 17175 Da). **E.** Tryptic MS/MS spectra confirm K6 as the site of isopeptide bond formation in the purified K6-linked diubiquitin sample. The peptide MQIFVK(GG)TLTGK contains two glycine residues from the donor ubiquitin attached to the acceptor ubiquitin. **F.** Tryptic MS/MS spectra of the AK(GG)IQDKEGIPPDQQR confirm K29 as the site of isopeptide bond formation in the purified K29-linked diubiquitin sample.

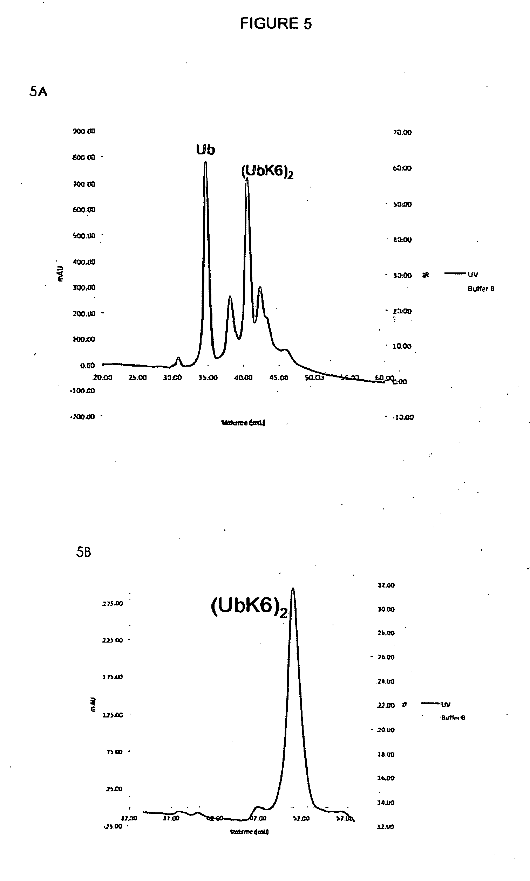

**Figure 5** **-** Purification of (UbK6)₂. **A.** First round of ion exchange chromatography on crude refolded (UbK6)₂. Buffer A is NH₄OAc pH 4.5. Buffer B is NH₄OAc pH 4.5, 1 M NaCl. **B.** Second round of ion exchange chromatography yielding pure K6-linked diubiquitin. Buffer A is NH₄OAc pH 4.5. Buffer B is NH₄OAc pH 4.5, 1 M NaCl.

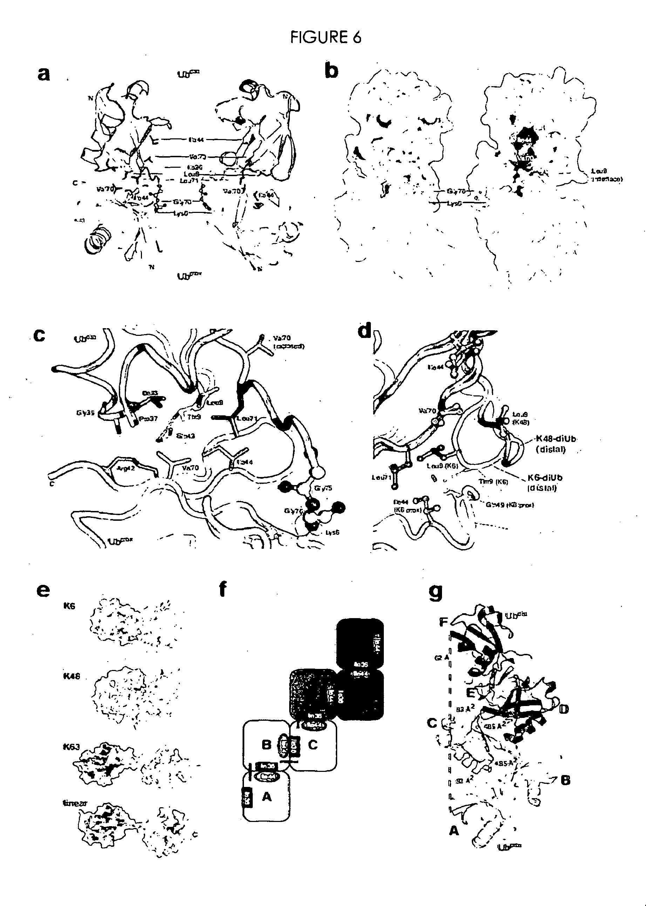

**Figure 6** **-** Structure of K6-linked diubiquitin **A.** Two views of the compact K6-linked diubiquitin molecules derived from the crystal structure, in cartoon representation with key residues shown in ball-and-stick representation. The distal molecule (yellow) is linked via its C-terminus to Lys6 of the proximal moiety (orange). Hydrophobic surface residues are colored in blue (Ile44, Val70) and green (Leu8), respectively, and the N- and C-termini of the ubiquitin molecules are indicated. **B.** K6-linked diubiquitin in the same views as in **A** are shown in surface representation. The exposed Ile44, Val70 of the distal molecule is indicated in blue. **C.** Close-up view of the ubiquitin-ubiquitin interface in a K6-linked dimer. The ubiquitin backbone colored as in **A** are shown as a thick ribbon, and interface residues are shown in stick-representation. Hydrophobic residues forming the proximal interface (Ile44, Val70, Arg42) are in blue and on the distal interface (Leu71, Leu8, Ile36) in green. An interface hydrogen bond between Gln49^{prox} and Thr9^{dist} is drawn as grey dotted line. **D.** The distal ubiquitin molecule from the structure of K48-linked diubiquitin (pink, pdb-id laar,⁴⁶) is superimposed on the distal ubiquitin from the K6-linked diubiquitin (yellow/orange, as in **A.** Leu8 commonly contributes to the hydrophobicity of the Ile44 patch in K48-linked diubiquitin and most ubiquitin complex structures. Conformational changes in the Leu8-loop in the K6-linked diubiquitin remove Leu8 from the Ile44-surface, to participate in the perpendicular Ile36 surface in the K6-interaction. **E.** Comparison of K6-, K48-, K63- and linear diubiquitin structures. **F.** Due to the asymmetric interface of K6-linked diubiquitin, propagation of the K6-contacts is possible. **G.** Modeling of a K6-linked ubiquitin hexamer in which proximal ubiquitin moieties of dimers are superimposed onto the distal ubiquitin successively. This reveals that elongated K6-linked chains may form helical filaments with a five-fold screw axis. No clashes between individual ubiquitin molecules, but additional interfaces to Ub+2 ad Ub-2 (83 Å² between molecules C : A, and C : E) in the chain can be formed in this arrangement of ubiquitin units (values shown for molecule C).

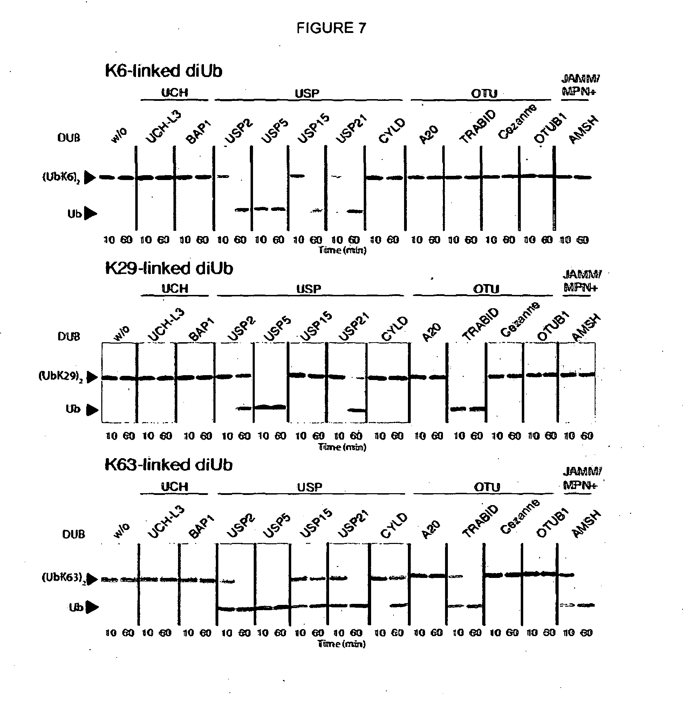

**Figure 7** **-** Profiling of DUB activity towards (UbK6)₂, (UbK29)₂ and (UbK63)₂. **A.** K6-linked diubiquitin (UbK6)₂ profiled against the deubiquitinase indicated above the gel. Samples analyzed by SDS-PAGE after 10 and 60 min. The deubiquitinase family is indicated above the gel. Deubiquitinase assays were carried out as previously described⁷. 3 µg of diubiquitin was incubated with the indicated deubiquitinase in a 30 µL reaction. The reactions were quenched and loaded on to SDS-PAGE to resolve diubiquitin from monoubiquitin (Ub), resulting from deubiquitinase-mediated cleavage. For the highly active USP2, USP5 and USP21 DUBs we used 0.2 µg of enzyme per reaction. We used 0.1 µg of Cezanne, as this is sufficient for complete hydrolysis of Kill-linked diubiquitin (A. Bremm, data not shown). For all other DUBs, 2 µg of enzyme was used. **B** and **C.** K29- and K63-linked diubiquitin respectively, profiled against the same deubiquitinases used for K6 experiments.

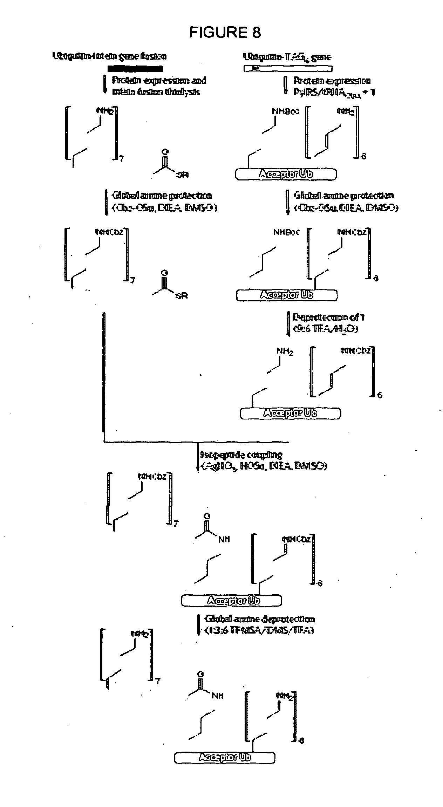

**Figure 8** **shows GOPAL strategy for site-specific isopeptide bond formation, exemplified for the synthesis of Lys6-linked diubiquitin. 1** is *N*ε*-*(*t-*butyloxycarbonyl)-L-lysine, shown in blue. Cbz-OSu is *N*-(benzyloxycarbonyl) succinimide, which reacts with the *N*ε-amine of lysine in proteins to give *N*ε-(benzyloxycarbonyl)-L-lysine. TFA is trifluoroacetic acid, DIEA is *N,N-*diisopropylethylamine, HOSu is *N*-(hydroxy)succinimide and TFMSA is trifluoromethanesulfonic acid.

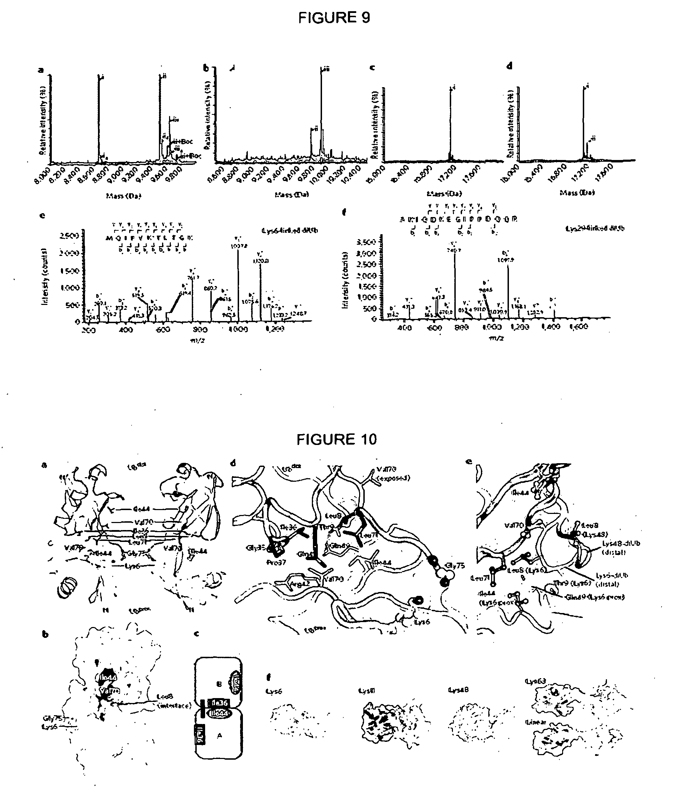

**Figure 9** **Synthesis and characterization of Lys6- and Lys29-linked ubiquitin. (a)** ESI-MS of the acceptor ubiquitin. In the green trace, ubiquitin with **1** genetically incorporated at position 6 (UbBocLys6) is "i" (observed mass = 8,665 Da; calculated mass = 8,665 Da). "ia" is the Na⁺ adduct. The blue trace is UbBocLys6 after chemical protection with Cbz and selective deprotection of the Boc protecting group of **1** at position 6. "ii" is UbLys6(Cbz₇), observed = 9,501.5 Da, calculated = 9,503 Da; "iia" is UbLys6(Cbz₇) + Na⁺; "iii" is UbLys6(Cbz₈), observed = 9,636.5 Da, calculated = 9,637 Da; "iiia" is UbLys6(Cbz₈) + Na⁺. "ii+Boc" and "iii+Boc" correspond to remaining traces of UbBocLys6(Cbz₇) and UbBocLys6(Cbz₈), respectively. (**b**) Purified ubiquitin-MES thioester (UbSR, orange trace, "i," observed = 8,690 Da, calculated = 8,689 Da). The blue trace shows UbSR after Cbz protection. "ii" is UbSR(Cbz₈), observed = 9,760 Da, calculated = 9,761 Da; "iii" is UbSR(Cbz₉), observed = 9,895 Da, calculated = 9,895 Da. (**c**) ESI-MS analysis of purified Lys6-linked diubiquitin (UbLys6₂) demonstrates the formation of an isopeptide bond (observed = 17,113 Da, calculated = 17,112 Da). (**d**) ESI-MS analysis of purified Lys29-linked diubiquitin (UbLys29₂). "i" is the formation of an isopeptide bond (observed = 17,111 Da, calculated = 17,112 Da); "ii" is the Na⁺, K⁺ adduct (observed = 17,174 Da, calculated = 17,172 Da). (**e,f**) Tryptic MS/MS spectra confirm Lys6 and Lys29 as the site of isopeptide bond formation in the purified Lys6 and Lys29 ligation samples, respectively, diUb, diubiquitin.
**Figure 10** **Structure of Lys6-linked diubiquitin. (a)** Two views of Lys6-linked diubiquitin, with the distal molecule in yellow and the proximal moiety in orange. Hydrophobic surface residues are colored in blue (Ile44, Val70) and green (Leu8, Ile36, Leu71). The isopeptide linkage is flexible, and Gly76 is disordered (see **Supplementary** **Fig. 3a****). (b)** Lys6-linked diubiquitin shown in surface representation. The exposed Ile44 and Val70 of the distal molecule are indicated in blue. (**c**) Schematic drawing of asymmetric Lys6-linked diubiquitin. (**d**) Close-up view of the Lys6-linked diubiquitin interface. Residues forming the proximal interface are in blue, and residues forming the distal interface are in green. An interface hydrogen bond between Gln49^{prox} and Thr9^{dist} is drawn as a gray dotted line. (**e**) The distal ubiquitin molecule from the structure of Lys48-linked diubiquitin (gray, PDB: laar⁴¹) is superimposed on the distal ubiquitin from the Lys6-linked diubiquitin (yellow and orange, as in **a**). Leu8 of the Ile44 patch in Lys48-linked diubiquitin undergoes a conformational change in Lys6-linked diubiquitin to participate in the Ile36 surface and in the Lys6-dimer interface. (**f**) Comparison of Lys6-, Lys11- (PDB: 2xew¹¹), Lys48- (PDB: 1aar⁴¹) and Lys63-linked (PDB: 2jf5; ref. 7) diubiquitin and linear diubiquitin (PDB: 2w9n⁷) structures. The proximal molecule is shown in a lighter color, and Ile44, Val70 and Leu8 are colored blue.

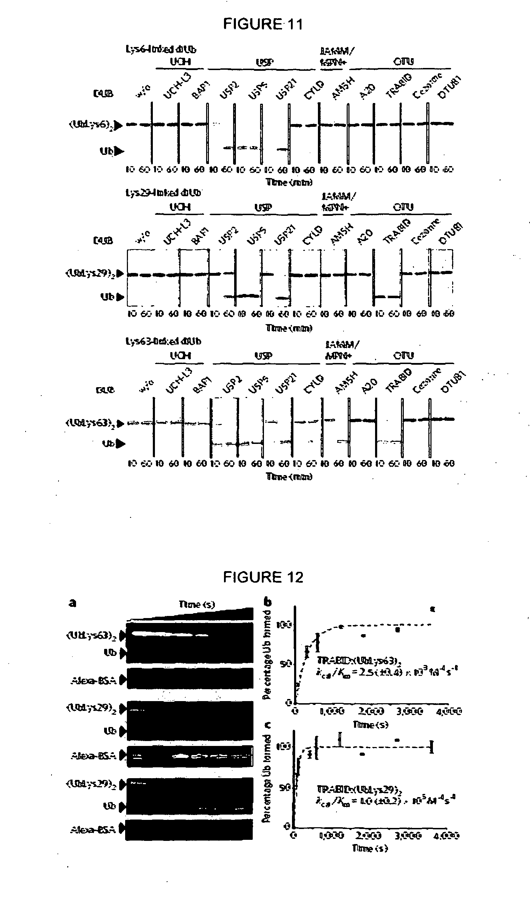

**Figure 11** **Profiling of deubiquitinase activity toward (UbLys6)₂, (UbLys29)₂ and (UbLys63)₂.** Each linkage was profiled against the deubiquitinase (DUB) indicated above the gel. Samples were analyzed by SDS-PAGE and silver staining after 10 and 60 min. The deubiquitinase family is indicated above the deubiquitinase. Deubiquitinase assays were carried out as described in the Supplementary Methods. Full gels in Supplementary Figure 4.
**Figure 12** **The specificity constant of TRABID is 40-fold higher on (UbLys29)₂ than on (UbLys63)₂ as determined by quantitative western blot. (a)** Representative quantitative western blots. Loading was normalized using the fluorescence of Alexa-BSA, which was included in the reaction. The upper two blots are with 1.3 µM TRABID, and the bottom blot is with 130 nM TRABID. The 0 time point lane for TRABID at 1.3 µM on Lys29 linkages is from the same gel as the other time points, but intervening, irrelevant lanes have been removed. **(b,c)** The progress curves for at least three independent trials of (UbLys63)₂ and (UbLys29)₂ cleavage with TRABID were fit, as described in the **Supplementary Methods,** to obtain specificity constants. Error bars represent the standard error. Full gels in **Supplementary** **Figure 7****.** Ub, ubiquitin.

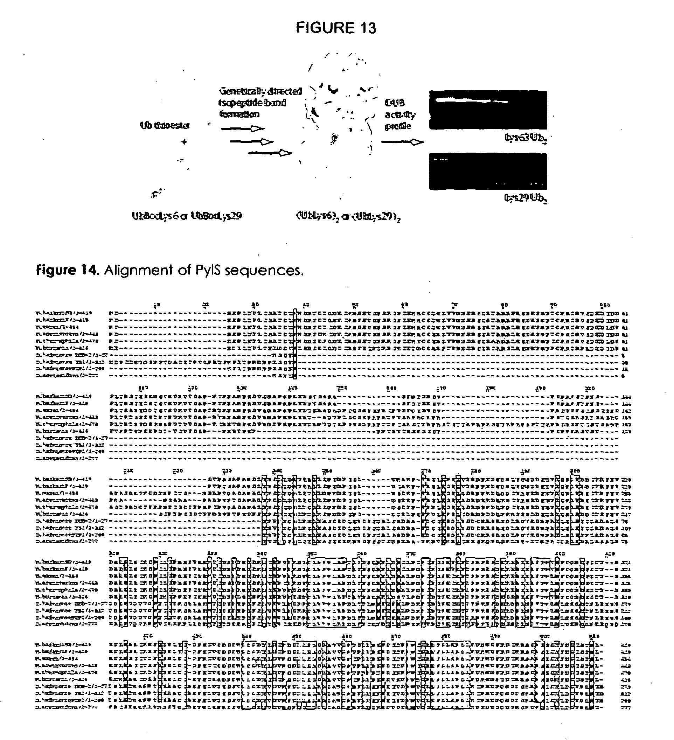

Figure 13 shows a diagram and photographs.
Figure 14 shows alignment of PylS sequences.

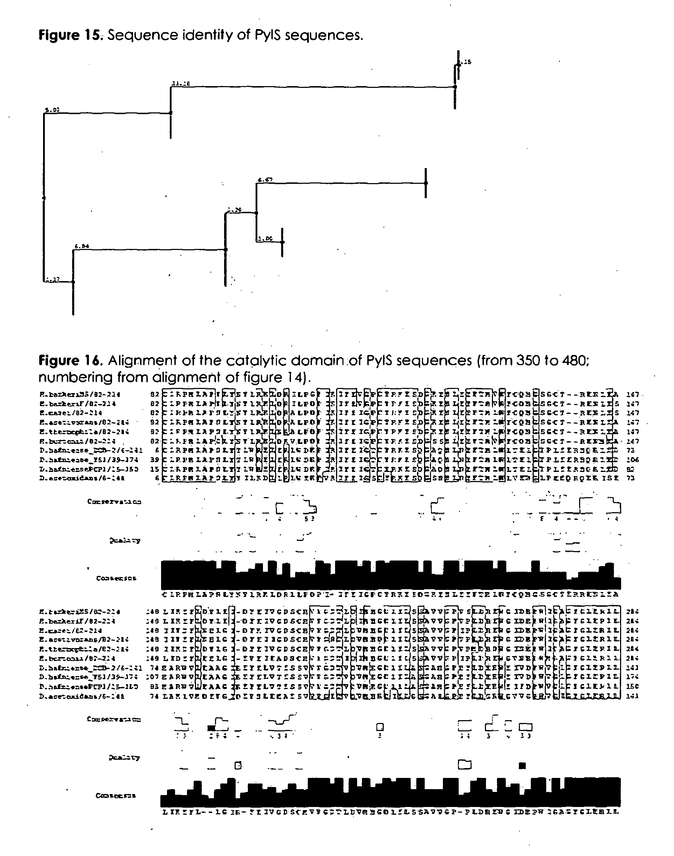

Figure 15 shows sequence identity of PylS sequences.
Figure 16 shows alignment of the catalytic domain of PylS sequences (from 350 to 480;
numbering from alignment of figure 14).

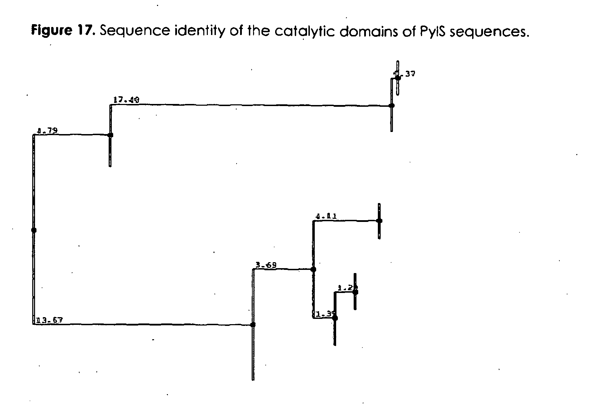

Figure 17 shows sequence identity of the catalytic domains of PylS sequences.

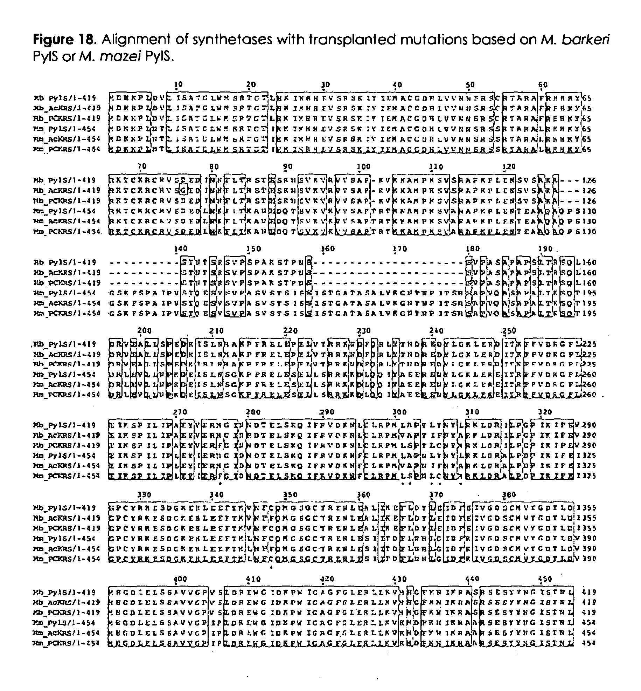

Figure 18 shows alignment of synthetases with transplanted mutations based on *M.barkeri* PylS or *M.mazei* PylS. The red asterisks indicate the mutated positions.

### Detailed Description of the Invention

It is vital to the invention that the protection groups on the target lysine and on other lysine(s) in the polypeptide are different. It is through the chemical differences of these protection groups that the differential deprotection chemistry permits specific or selective modification of the target residue by enabling its specific or selective deprotection and therefore modification.

Suitably the desired reaction in step (d) is carried out on the ε-amino group of the lysine residue.

In another preferred embodiment, the other lysine side chains to be protected in step (b) are also the ε-amino group. In such a case, the method above also applies to the terminal amino group of the polypeptide chain.

In the method according to the invention, said genetic incorporation preferably uses an orthogonal or expanded genetic code, in which one or more specific orthogonal codons have been allocated to encode the specific lysine residue with the lysine side group chain protected so that it can be genetically incorporated by using an orthogonal tRNA synthetase/tRNA pair. The orthogonal tRNA synthetase/tRNA pair can in principle be any such pair capable of charging the tRNA with the protected lysine and capable of incorporating that protected lysine into the polypeptide chain in response to the orthogonal codon.

The orthogonal codon may be the orthogonal codon amber, ochre, opal or a quadruplet codon. The codon simply has to correspond to the orthogonal tRNA which will be used to carry the protected lysine molecule. Preferably the orthogonal codon is amber.

It should be noted that the specific examples shown herein have used the amber codon and the corresponding tRNA/tRNA synthetase. As noted above, these may be varied. Alternatively, in order to use other codons without going to the trouble of using or selecting alternative tRNA/tRNA synthetase pairs capable of working with the protected lysine, the anticodon region of the tRNA may simply be swapped for the desired anticodon region for the codon of choice. The anticodon region is not involved in the charging or incorporation functions of the tRNA nor recognition by the tRNA synthetase so such swaps are entirely within the ambit of the skilled operator.

Thus alternative orthogonal tRNA synthetase/tRNA pairs may be used if desired.

Preferably the orthogonal synthetase/tRNA pair are *Methanosarcina barkeri* MS pyrrolysine tRNA synthetase (*Mb*PyIRS) and its cognate amber suppressor tRNA (MbtRNA_{CUA}).

The *Methanosarcina barkeri* PylT gene encodes the *Mb*tRNA_{CUA} tRNA. The *Methanosarcina bar*keri PylS gene encodes the *Mb*PyIRS tRNA synthetase protein. When particular amino acid residues are referred to using numeric addresses, the numbering is taken using *Mb*PyIRS (*Methanosarcina barkeri* pyrrolysyl-tRNA synthetase) amino acid sequence as the reference sequence (i.e. as encoded by the publicly available wild type *Methanosarcina barkeri* PylS gene Accession number Q46E77) :
MDKKPLDVLI SATGLWMSRT GTLHKIKHYE VSRSKIYIEM ACGDHLVVNN SRSCRTARAF RHHKYRKTCK RCRVSDEDIN NFLTRSTEGK TSVKVKVVSA PKVKKAMPKS VSRAPKPLEN PVSAKASTDT SRSVPSPAKS TPNSPVPTSA PAPSLTRSQL DRVEALLSPE DKISLNIAKP FRELESELVT RRKNDFQRLY TNDREDYLGK LERDITKFFV DRDFLEIKSP ILIPAEYVER MGINNDTELS KQIFRVDKNL CLRPMLAPTL YNYLRKLDRI LPDPIKIFEV GPCYRKESDG KEHLEEFTMV NFCQMGSGCT RENLESLIKE FLDYLEIDFE IVGDSCMVYG DTLDIMHGDL ELSSAVVGPV PLDREWGIDK PWIGAGFGLE RLLKVMHGFK NIKRASRSES YYNGISTNL.

Said sequence has been annotated here below as SEQ ID NO.1.

If required, the person skilled in the art may adapt MbPyIRS tRNA synthetase protein by mutating it so as to optimise for the lysine species with particular protected side chains to be used. The need for mutation depends on the lysine reisdue and protection/caging group used. An example where the MbPyIRS tRNA synthetase does not need to be mutated is when the lysine residues with protected side chains used in step (a) are Nε-(t-butyloxycarbonyl)-L-lysine. An example where the MbPyIRS tRNA synthetase may need to be mutated is when the lysine side group chain in step (a) is protected by a larger chemical group such as a photolabile caging group.

Such mutation may be carried out by introducing mutations at one or more of the following positions in the MbPyIRS tRNA synthetase: M241, A267, Y271, L274 and C313. Preferably the mutations may comprise M241 F, A267S, Y271 C and L274M.

### tRNA Synthetases

The tRNA synthetase used in the invention may be varied. Although specific tRNA synthetase sequences may have been used in the examples, the invention is not intended to be confined only to those examples.

In principle any tRNA synthetase which provides the same tRNA charging (aminoacylation) function can be employed in the invention.

For example the tRNA synthetase may be from any suitable species such as from archea, for example from *Methanosarcina barkeri* MS; *Methanosarcina barkeri* str. Fusaro; *Methanosarcina mazei* Gol; *Methanosarcina acetivorans* C2A; *Methanosarcina thermophila;* or *Methanococcoides burtonii.* Alternatively the the tRNA synthetase may be from bacteria, for example from *Desulfitobacferium hafniense* DCB-2; *Desulfitobacterium hafniense* Y51; *Desulfitobacterium hafniense* PCP1; *Desulfotomaculum acetoxidans* DSM 771.

Exemplary sequences from these organisms are the publically available sequences. The following examples are provided as exemplary sequences for pyrrolysine tRNA synthetases:
*>M.barkeriMS*/1-419/
*Methanosarcina barkeri* MS
VERSION Q6WRH6.1 GI:74501411
*>M. barkeriF*/*1-419*/
*Methanosarcina barkeri* str. Fusaro
VERSION YP_304395.1 GI:73668380
*>M.mazei*/*1-454*
*Methanosarcina mazei* Gol
VERSION NP_633469.1 GI:21227547
>M.acetivorans/1-443
*Methanosarcina acetivorans* C2A
VERSION NP_615128.2 GI: 161484944
*>M.thermophila*/1-478
*Methanosarcina thermophila,* VERSION DQ017250.1 GI:67773308
*>M.burfonii*/*1-416*
*Methanococcoides burfonii* DSM 6242, VERSION YP_566710.1 GI:91774018
*>D.hafniense*_DCB-2/1-279
*Desulfitobacferium hafniense* DCB-2
VERSION YP_002461289.1 GI:219670854
>*D.hafniense*_Y51/1-312
*Desulfitobacterium hafniense* Y51
VERSION YP_521192.1 GI:89897705
>*D.hafniensePCP* 1/1-288
*Desulfitobacterium hafniense*
VERSION AY692340.1 GI:53771772
*>D.acetoxidans*/*1* -277
*Desulfotomaculum acetoxidans* DSM 771
VERSION YP_003189614.1 GI:258513392

When the particular tRNA charging (aminoacylation) function has been provided by mutating the tRNA synthetase, then it may not be appropriate to simply use another wild-type tRNA sequence, for example one selected from the above. In this scenario, it will be important to preserve the same tRNA charging (aminoacylation) function. This is accomplished by transferring the mutation(s) in the exemplary tRNA synthetase into an alternate tRNA synthetase backbone, such as one selected from the above.

In this way it should be possible to transfer selected mutations to corresponding tRNA synthetase sequences such as corresponding pylS sequences from other organisms beyond exemplary *M.barkeri* and/or *M.mazei* sequences.

Target tRNA synthetase proteins/backbones, may be selected by alignment to known tRNA synthetases such as exemplary *M.barkeri* and/or *M.mazei* sequences.

This subject is now illustrated by reference to the pylS (pyrrolysine tRNA synthetase) sequences but the principles apply equally to the particular tRNA synthetase of interest.

For example, figure 14 provides an alignment of all PylS sequences. These can have a low overall % sequence identity. Thus it is important to study the sequence such as by aligning the sequence to known tRNA synthetases (rather than simply to use a low sequence identity score) to ensure that the sequence being used is indeed a tRNA synthetase.

Thus suitably when sequence identity is being considered, suitably it is considered across the tRNA synthetases as in figure 14. Suitably the % identity may be as defined from figure 14. Figure 15 shows a diagram of sequence identities between the tRNA synthetases. Suitably the % identity may be as defined from figure 15.

It may be useful to focus on the catalytic region. Figure 16 aligns just the catalytic regions. The aim of this is to provide a tRNA catalytic region from which a high % identity can be defined to capture/identify backbone scaffolds suitable for accepting mutations transplanted in order to produce the same tRNA charging (aminoacylation) function, for example new or unnatural amino acid recognition.

Thus suitably when sequence identity is being considered, suitably it is considered across the catalytic region as in figure 16. Suitably the % identity may be as defined from figure 16. Figure 17 shows a diagram of sequence identities between the catalytic regions. Suitably the % identity may be as defined from figure 17.

'Transferring' or 'transplanting' mutations onto an alternate tRNA synthetase backbone can be accomplished by site directed mutagenesis of a nucleotide sequence encoding the tRNA synthetase backbone. This technique is well known in the art. Essentially the backbone pylS sequence is selected (for example using the active site alignment discussed above) and the selected mutations are transferred to (i.e. made in) the corresponding/homologous positions.

When particular amino acid residues are referred to using numeric addresses, unless otherwise apparent, the numbering is taken using MbPylRS (*Methanosarcina barkeri* pyrrolysyl-tRNA synthetase) amino acid sequence as the reference sequence (i.e. as encoded by the publicly available wild type *Methanosarcina barkeri* PylS gene Accession number Q46E77):
MDKKPLDVLI SATGLWMSRT GTLHKIKHYE VSRSKIYIEM ACGDHLVVNN SRSCRTARAF RHHKYRKTCK RCRVSDEDIN NFLTRSTEGK TSVKVKVVSA PKVKKAMPKS VSRAPKPLEN PVSAKASTDT SRSVPSPAKS TPNSPVPTSA PAPSLTRSQL DRVEALLSPE DKISLNIAKP FRELESELVT RRKNDFQRLY TNDREDYLGK LERDITKFFV DRDFLEIKSP ILIPAEYVER MGINNDTELS KQIFRVDKNL CLRPMLAPTL YNYLRKLDRI LPDPIKIFEV GPCYRKESDG KEHLEEFTMV NFCQMGSGCT RENLESLIKE FLDYLEIDFE IVGDSCMVYG DTLDIMHGDL ELSSAVVGPV PLDREWGIDK PWIGAGFGLE RLLKVMHGFK NIKRASRSES YYNGISTNL

This is to be used as is well understood in the art to locate the residue of interest. This is not always a strict counting exercise - attention must be paid to the context or alignment. For example, if the protein of interest is of a slightly different length, then location of the correct residue in that sequence corresponding to (for example) L266 may require the sequences to be aligned and the equivalent or corresponding residue picked, rather than simply taking the 266th residue of the sequence of interest. This is well within the ambit of the skilled reader.

Notation for mutations used herein is the standard in the art. For example L266M means that the amino acid corresponding to L at position 266 of the wild type sequence is replaced with M.

The transplantation of mutations between alternate tRNA backbones is now illustrated with reference to exemplary *M.barkeri* and *M.mazei* sequences, but the same principles apply equally to transplantation onto or from other backbones.

For example Mb AcKRS is an engineered synthetase for the incorporation of AcK
Parental protein/backbone: *M. barkeri* PylS
Mutations: L266V, L270I, Y271F, L274A, C317F

Mb PCKRS: engineered synthetase for the incorporation of PCK
Parental protein/backbone: *M*. *barkeri* PylS
Mutations: M241 F, A267S, Y271C, L274M

Synthetases with the same substrate specificities can be obtained by transplanting these mutations into *M. mazei* PylS. The sequence homology of the two synthetases can be seen in figure 18. Thus the following synthetases may be generated by transplantation of the mutations from the Mb backbone onto the Mm tRNA backbone: Mm AcKRS introducing mutations L301V, L305I, Y306F, L309A, C348F into *M. mazei* PylS, and
Mm PCKRS introducing mutations M276F, A302S, Y306C, L309M into *M. mazei* PylS.

Full length sequences of these exemplary transplanted mutation synthetases are given below.
>Mb_PylS/1-419
>Mb_AcKRS/1-419
>Mb_PCKRS/1-419
>Mm_PylS/1-454
>Mm_AcKRS/1-454
>Mm_PCKRS/1-454

The same principle applies equally to other mutations and/or to other backbones. Transplanted polypeptides produced in this manner should advantageously be tested to ensure that the desired function/substrate specificities have been preserved.

Polynucleotides encoding the polypeptide of interest for the method described above can be incorporated into a recombinant replicable vector. The vector may be used to replicate the nucleic acid in a compatible host cell. Thus further disclosed herein is a method of making polynucleotides by introducing a polynucleotide into a replicable vector, introducing the vector into a compatible host cell, and growing the host cell under conditions which bring about replication of the vector. The vector may be recovered from the host cell. Suitable host cells include bacteria such as *E. coli.*

Preferably, a polynucleotide in a vector is operably linked to a control sequence that is capable of providing for the expression of the coding sequence by the host cell, i.e. the vector is an expression vector. The term "operably linked" means that the components described are in a relationship permitting them to function in their intended manner. A regulatory sequence "operably linked" to a coding sequence is ligated in such a way that expression of the coding sequence is achieved under condition compatible with the control sequences.

Vectors may be transformed or transfected into a suitable host cell as described to provide for expression of a protein of the invention. This process may comprise culturing a host cell transformed with an expression vector as described above under conditions to provide for expression by the vector of a coding sequence encoding the protein, and optionally recovering the expressed protein. The vectors may be for example, plasmid or virus vectors provided with an origin of replication, optionally a promoter for the expression of the said polynucleotide and optionally a regulator of the promoter. The vectors may contain one or more selectable marker genes, for example an ampicillin resistance gene in the case of a bacterial plasmid. Vectors may be used, for example, to transfect or transform a host cell.

Control sequences operably linked to sequences encoding the protein of the invention include promoters/enhancers and other expression regulation signals. These control sequences may be selected to be compatible with the host cell for which the expression vector is designed to be used in. The term promoter is well-known in the art and encompasses nucleic acid regions ranging in size and complexity from minimal promoters to promoters including upstream elements and enhancers.

The target lysine is protected by a protecting group (e.g. step (a) of the method). Said protecting group is different from the protecting group used to protect the further lysine(s) (e.g. step (b) of the method). The method of deprotecting used to selectively remove the protecting group from the target lysine in step (c) of the method must be performed so as NOT to deprotect the further lysine(s) at the same time. Chemical protecting agents for lysine side chains are varied and can be chosen by the person skilled in the art depending on the type of deprotection methods to be used. However said protecting agent is suitably chosen so as to allow the lysine residue to be incorporated genetically and thus allow it to be incorporated by an orthogonal synthetase/tRNA pair in a cell.

Suitably the protecting agent used in step (a) is as described herein. More suitably, the lysine amino acid with protecting agent to be used in step (a) is Nε-(t-butyloxycorbonyl)-L-lysine.

Another embodiment employs a lysine amino acid protected with a photo-labile caging group. This has the advantage of permitting photo-decaging (deprotection) which can be easier than chemical deprotection.

The further lysine residues and, optionally the N-terminal amino group, have their side chains protected to allow for the specific modification of the target lysine. This is advantageously accomplished using a reaction where the protecting group can reach, or at least approach, saturation (100%) of the further lysine residues present in the polypeptidic chain.

It is advantageous to use a polypeptide which can be easily denatured and renatured without losing its properties as a protein. In this regard it may be advantageous if the polypeptide according to the invention is a small protein. It is further advantageous for the polypeptide to have few or no post-translational modifications such as for example glycosylation.

Exemplary polypeptides to be modified include histones and/or small transcription factors. Suitably the polypeptide to be modified is not too large. By not too large is meant suitably not more than a few hundred amino acids long; suitably 400 amino acids or fewer, more suitably approximately 300 amino acids or fewer. Multidomain proteins are less attractive for modification because of the increased chances of affecting interactions with other domain(s) (e.g. other members of a multiprotein complex).

Suitably the protection groups used may be as described herein. More suitably the protecting agent is N-(benzyloxycarbonyloxy) succinimide (Cbz-Osu). Suitably this may be used in basic DMSO.

Alternative or additional protecting groups and their manipulation (such as the chemistry for their addition / removal are described below.

BocLys is described in "Genetic incorporation of *N*ε-(*t*-butyloxycarbonyl)-L-lysine (BocLys)" (YANAGISAWA, T., ISHII, R., FUKUNAGA, R., KOBAYASHI, T., SAKAMOTO, K. & YOKOYAMA, S. (2008) Multistep engineering of pyrrolysyl-tRNA synthetase to genetically encode N(epsilon)-(o-azidobenzyloxycarbonyl) lysine for site-specific protein modification. Chem Biol, 15, 1187-97)
Off - trifluoroacetic acid (TFA) 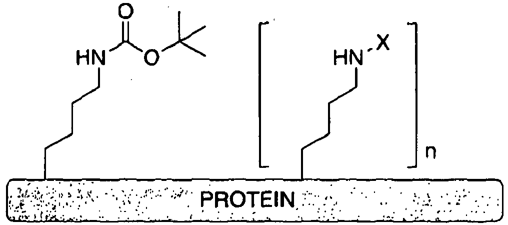 Orthogonal protecting groups that can be mildly attached to protein amines where X= 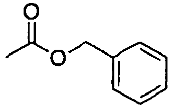
1. Benzyloxycarbonyl (Cbz or Z)
   On -N*-*(benzyloxycarbonyloxy)-succinimide
   (KAWAKAMI, T., HASEGAWA, K., TERUYA, K., AKAJI, K., HORIUCHI, M., INAGAKI, F., KURIHARA, Y., UESUGI, S. & AIMOTO, S. (2001) Polypeptide synthesis using an expressed peptide as a building block for condensation with a peptide thioester: application to the synthesis of phosphorylated p21Max protein(1-101). J Pept Sci, 7, 474-87)
   Off - HF/DMS, TFMSA/TFA/DMS, catalytic hydrogenation
   (TAM, J., HEATH, W. & MERRIFIELD, R. (1983) SN2 DEPROTECTION OF SYNTHETIC PEPTIDES WITH A LOW CONCENTRATION OF HF IN DIMETHYL SULFIDE - EVIDENCE AND APPLICATION IN PEPTIDE-SYNTHESIS. Journal of the American Chemical Society, 105, 6442-6455)
   (TAM, J. P., HEATH, W. F. & MERRIFIELD, R. B. (1986) Mechanisms for the removal of benzyl protecting groups in synthetic peptides by trifluoromethanesulfonic acid trifluoroacetic-acid dimethyl sulfide. Journal of the American Chemical Society, 108, 5242-5251)
   Greene's Protective Groups in Organic Synthesis 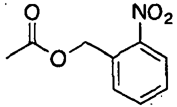
2. *o*-nitrobenzyloxycarbonyl
   On - *N*-(o-nitrobenzyloxycarbonyloxy)-succinimide
   Off - *hv*∼365 nm, aq. Na₂S₂O₄ (1,4-elimination) 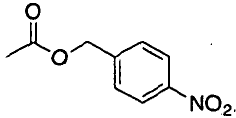
3. *p*-nitrobenzyloxycarbonyl
   On - *N*-(o-nitrobenzyloxycarbonyloxy)-succinimide
   Off - aq. Na₂S₂O₄ (1,6-elimination) 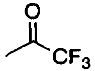
4. Trifluoroacetyl (TFAc)
   On - Trifluoroaceticanhydride
   Off - K₂CO₃ / aqeous MeOH, pH > 9
   Greene's Protective Groups in Organic Synthesis 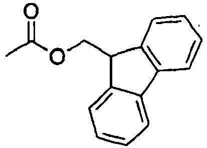
5. Fluorenylmethoxycarbonyl (Fmoc)
   On - *N*-(Fluorenylmethoxycarbonyloxy)-succinimide
   Off - Base
   Can be removed by any primary amine therefore appropriate attention must be paid to conditions when using this protecting group.
   Greene's Protective Groups in Organic Synthesis 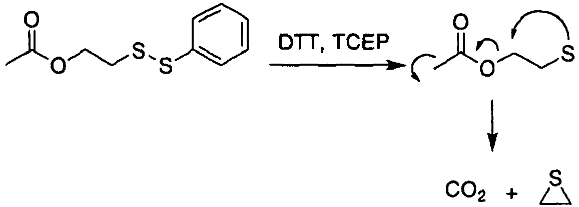
6. Aryldithioethyloxycarbonyl (Ardec)
   (LAPEYRE, M., LEPRINCE, J., MASSONNEAU'LD, M., OULYADI, H., RENARD, P., ROMIEU, A., TURCATTI, G. & VAUDRY, H. (2006) Aryldithioethyloxycarbonyl (Ardec): A new family of amine protecting groups removable under mild reducing conditions and their applications to peptide synthesis. Chem-Eur J, 12,3655-3671)
   On - *N*-(Aryldithioethyloxycarbonyl)-succinimide
   Off - mild reduction (TCEP, DTT, BME)
Genetic incorporation of *N*ε-(trifluoroacetyl)-L-lysine (TFAcLys) 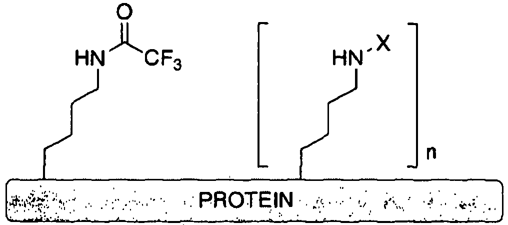 Off - K₂CO₃ / aqeous MeOH, pH > 9
Orthogonal protecting groups that can be mildly attached to protein amines where X= 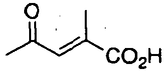
1. Citraconyl
   On - citraconic anhydride/aqueous buffer pH 8
   Off - pH 3 - 4
   (BLAKE, J. & LI, C. (1981) New segment-coupling method for peptide synthesis in aqueous solution: application to synthesis of human [Gly17]-beta-endorphin. Proc Natl Acad Sci USA, 78, 4055-8) 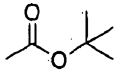
2. *t*-butyloxycarbonyl (Boc)
   On - *N*-(*t*-butyloxycarbonyloxy)-succinimide
   Off -TFA
Cbz, photocages and Ardec may also be used with genetically encoded TFAc protection.
Genetic incorporation of *N*ε-(*o*-nitropiperonyloxycarbonyl)-L-lysine (ONPOC) (GAUTIER, A., NGUYEN, D. P., LUSIC, H., AN, W., DEITERS, A. & CHIN, J. W. (2010) Genetically encoded photocontrol of protein localization in mammalian cells. Journal of the American Chemical Society, 132, 4086-8)
Off- h *v ∼365* nm 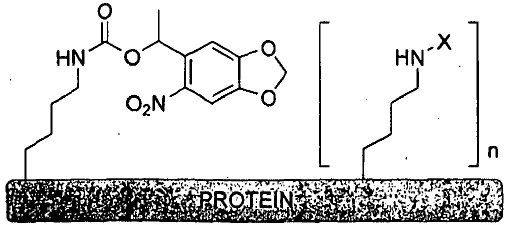 Regarded as compatible with base cleavable and mild acid cleavable protecting groups. Ardec may also find application in the invention.
Genetic incorporation of *N*ε-(allyoxycarbonyl)-L-lysine (AllocLys) (YANAGISAWA, T., ISHII, R., FUKUNAGA, R., KOBAYASHI, T., SAKAMOTO. K. & YOKOYAMA, S. (2008) Multistep engineering of pyrrolysyl-tRNA synthetase to genetically encode N(epsilon)-(o-azidobenzyloxycarbonyl) lysine for site-specific protein modification. Chem Biol, 15, 1187-97) 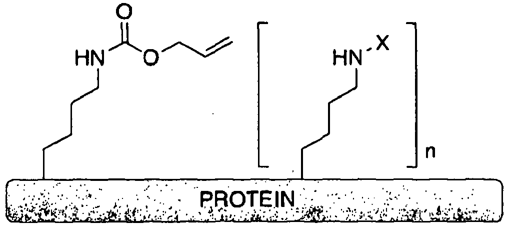 Off - Ni(CO)₄, (PdPh₃P)₄/Bu₃SnH/AcOH

This has the advantage of being orthogonal with everything except catalytic deprotection and probably not HF/TFMSA.

In another embodiment the further lysine side chains may be protected with a photo-labile caged lysine. In this embodiment when the target lysine is also protected by a photolabile group, the protection of the target lysine(s) should be by a photolabile group released at a frequency of radiation which will not release the second protecting group.

The present method enables targeting of one or more specific lysines present within a polypeptide chain for a specific reaction through recombinant techniques and chemoselective protein chemistry. Suitably the method is an *in vitro* method.

An advantage of the invention is the provision of a method to study the effect of lysine modifications in polypeptides and proteins. One example of such a modification dimethylation of lysine(s). This finds application in studying the effect of dimethylation of lysines in proteins.

In another preferred example, the reaction in step (d) is covalently linking the lysine residue to another protein. As the specific lysine side chain presents an ε-amino after deprotection in step (c), it is preferable that the reaction be a peptide bond formation. In such cases, the other protein may also present lysine side chains and thus it is preferable if the other protein be protected in the same manner in step (b) together with the other lysine residues of the polypeptidic chain. Suitably the whole modified polypeptide is deprotected after synthesis.

As is mentioned above, the invention is useful in ubiquitination of polypeptide(s) and/or the study of same. Thus it is preferable if the polypeptide and/or other polypeptide comprise ubiquitin. Ubiquitin is a small protein that is easily denatured and renatured, allowing for its ease of production by recombination and selective protein chemistry according to the method of the present invention.

Thus the present method is a powerful tool that allows ubiquitination in a specific manner of any protein. When the effects of such ubquitination are known, such as for example by linking a protein to a polyubiquitin linked by K48 of the ubiquitin polypeptide, this can be helpful in studying their proteosomal degradation.

In another example, the method can be repeated to allow one to link several proteins together. In the case that both the polypeptidic chain and the protein are ubiquitin, it is possible to use the invention to produce ubiquitin chains, for example homogenously and/or heterogeneously linked.

Thus also disclosed herein are the resulting polypeptidic chains that are obtainable from the methods according to the invention, suitably a polypeptidic chain linked specifically to a protein by an isopeptide bond. One such example are ubiquitinated proteins, whereby the reaction in step (d) is to link the ubiquitin to another protein by peptide bond formation.

Another such example are the homogenously linked ubiquitin chains obtainable by the method. As shown below, a homogenously linked ubiquitin chain has been obtained according to the method where the covalent link is an isopeptide bond between a lysine amino acid residue at position 6 or 29 and the C-terminus of another ubiquitin polypeptide. It is to be understood that the chain can be continued by further homogenous linkages, further obtainable by the method according to the invention.

Also disclosed herein is the homogenously linked ubiquitin obtained according to the method of the invention where the covalent link is an isopeptide bond between a lysine amino acid residue at position 6 and the C-terminus of another ubiquitin polypeptide. The linkages can be continued for more than 2 links.

Said ubiquitin chain can be used as a medicament. It can be used in activating or promoting a response to DNA damage. The chains have been shown to be linked to the BRCA1/Bard1 E3 ligase complex and thus the ubiquitin chains can be used in preventing or treating cancer, preferably where the cancer is early-onset breast or ovarian cancer.

As such, the ubiquitin chains can be treated as an oncological medicament and can be used in pharmaceutical compositions and administered by means well known in the art in the filed of oncological pharmacy.

The following non-limiting examples are illustrative of the present invention:
The specific formation of an isopeptide bond between the C-terminus of one ubiquitin (donor) and the amine of a specific lysine residue in another ubiquitin (acceptor) requires these functional groups to be differentiated from the other carboxylic acids and amines in both ubiquitin molecules (Figure 1). It was done by applying the method according to the invention to the acceptor ubiquitin and step (b) to the donor ubiquitin. To exemplify the generality of this approach it was planned to make ubiquitin dimers linked through a specific isopeptide bond between the C-terminus of the donor ubiquitin and an ε-amino group of a lysine residue on the acceptor ubiquitin, (either K6 or K29). Since each ubiquitin contains 7 lysines this system provides a stringent test of the specificity of our approach.

### Example 1 - Preparation of an acceptor ubiauith according to the method of the invention

The present Example is the preparation of a ubiquitin molecule according to the method of the invention to be an acceptor ubiquitin (for a successive isopeptide bond later on). This method is represented partly by the right hand side of Figure 1.

The protocols used were

### Cloning of acceptor ubiquitin (UbTAG6-His₆ and UbTAG29-His₆)

The human UBC ubiquitin gene was PCR amplified using a forward 5'-CG CGC GCC ATG GAG ATC TTC GTG AAG ACC CTG ACT GG-3' primer and the reverse 5'-GCC GGA TCT CCG CTC GAG TTA GTG GTG ATG ATG GTG ATG CCC ACC TCT GAG ACG GAG GAC-3' primer, that introduce Ncol and Xhol restriction sites as well as a C-terminal His₆ tag followed by a stop codon. The PCR product was digested with Ncol and XhoI and ligated into a similarly treated pCDF-*PylT* plasmid (which encodes *Mb*tRNACUA on an *Ipp* promoter and *rrnC* terminator and has a spectinomycin resistance maker^{20,42}). The forward primer forced a mutation of the second ubiquitin codon and as such, Quikchange mutagenesis was required to mutate the second ubiquitin residue back to Gln. A second round of Quikchange mutagenesis was then used to introduce a TAG codon at position K6 or K29. The final plasmids were named p*CDF-pylT-UbTAG6-His₆* and pCDF-*pylT-UbTAG29-His₆* respectively.

### Preparation of acceptor ubiquitin (UbBocK6 and UbBocK29)

BL21 (DE3) cells (Merck biosciences) containing pBKPylS (a kanamycin resistant plasmid encoding *M*bPylRS on an *E. coli* GlnRS promoter and terminator) and pCDF-*pylT*-UbTAG6-His₆ were grown to overnight (37°C, 230 rpm, in LB-KS: LB media containing 50 µg mL⁻¹ spectinomycin and 50 µg mL⁻¹ kanamycin). The culture was diluted 1:50 in to 2L of fresh LB-KS and incubated (37°C, 230 rpm). At OD₆₀₀=0.6,

a solution of 200 mM **1** (10 mL in 2 M aq. NaOH) was added to the cells whilst stirring vigorously and the culture was immediately neutralized with 5 M HCl (4 mL). After 30 min protein expression was induced by the addition of isopropyl-βD-thiogalactopyranoside to 0.5 mM. After incubation (37°C, 230 rpm, 3h) cells were harvested by centrifugation and resuspended in 50 mL ice-cold lysis buffer (20 mM Na₂HPO₄ pH 7.4, 25 mM imidazole) and frozen at -80°C until required. Cells were thawed on ice and lysozyme (0.5 mg mL⁻¹) and DNAseA (50 µg mL⁻¹) were added. After 30 min the cells were sonicated and clarified by centrifugation (39000 x g, 30 min). The clarified lysate was loaded, by gravity flow, onto a column containing Ni-NTA resin (3 mL, Qiagen). The resin was washed with lysis buffer (90 mL) and the protein eluted with elution buffer (20 mM Na₂HPO₄ pH 7.4, 250 mM imidazole). Fractions containing UbBocK6-His₆ were determined by SDS-PAGE and were pooled and concentrated to < 9 mL with an Amicon Ultra-15 3 kDa MWCO centrifugal filter device (Millipore). The sample was then dialyzed against 10 mM Tris pH 7.6 for 3 hours. 1 mM DTT was added to the UbBocK6-His₆ sample, followed by UCH-L3 at a final concentration of 15 µg mL⁻¹. The sample was incubated at 37 °C for 1 hour to remove the C-terminal His₆ tag. UbBocK6 was isolated by size-exclusion chromatography employing a HiLoad 26/60 Superdex 75 Prep Grade column (GE Life Sciences) at a flow rate of 2 mL min⁻¹. Fractions containing UbBocK6 were pooled and concentrated to 2.5 mL. The sample was desalted into H₂O using a PD-10 column (GE Life Sciences) and the elution was lyophilized yielding approximately 20 mg of UbBocK6. UbBocK29 was prepared the same way except that cells were transformed with pCDF-*pylT*-*UbTAG29*-*His₆*. The yield of UbBocK29 was 8 mg.

### Global protection of UbBocK6 and UbBocK29 with Cbz-OSu

Lyophilized UbBocK6 (20 mg, 2.3 µmol) was dissolved in DMSO (1.7 mL) followed by the addition of DIEA (67 µL). Whilst stirring, Cbz-OSu (4.81 mg, 19.3 µmol) was added and the reaction. After stirring for 2 h at 25 °C the reaction was transferred into cold ether (17 mL) and briefly vortexed. The precipitate was collected by centrifugation and the ether layer was discarded. The pellet was washed with ice-cold ether (17 mL) and then air-dried. For UbBocK29 this and subsequent procedures were repeated pro rata in account of the reduced expression yield of UbBocK29-His₆.

### Removal of Boc protecting group

The dry globally protected peptide, UbBocK6(Cbz₇₋₈), obtained from 10 mg of UbBocK6 was dissolved in cold 9:6 TFA:H₂O (1.28 mL) and incubated at 4 °C for 1 h. The selectively deprotected peptide, was then precipitated and washed with ice-cold ether (2 x 13 mL). The aqueous and ether layers were removed and the peptide was left to air dry.

### Results

To site-specifically install a protected lysine at position 6 of ubiquitin we took advantage of the *Methanosarcina barkeri MS* pyrrolysine tRNA synthetase (MbPyIRS) and its cognate amber suppressor tRNA (MbtRNA_{CUA})¹⁸, which directs the efficient incorporation of *N*ε-(*t-*butyloxycorbonyl)-L-lysine (**1**) into recombinant proteins in response to the amber codon in *E. coli*¹⁹⁻²¹. We created a ubiquitin expression construct in which the ubiquitin gene contains a TAG codon in place of the lysine codon at position 6, and is flanked by a 3' His₆ tag coding sequence (*UbTAG*6-*His*₆). We produced UbBocK6-His₆ (ubiquitin-his6 containing **1** at position) by expressing *UbTAG*6-*His*₆ in cells containing the MbPyIRS/ MbtRNA_{CUA}pair and **1** (2mM). UbBocK6-His₆ production was strictly dependent on the addition of **1**. UbBocK6-His₆ was purified by Ni-NTA chromatography and the His₆ tag was removed by treatment with ubiquitin C-terminal hydrolase-L3 (UCH-L3) to give UbBocK6 (Figure 2). The untagged UbBocK6 was then further purified by size-exclusion chromatography, desalted and lyophilized. The purified material was characterized by electrospray ionization mass spectrometry (ESI-MS) (Figure 3A). This procedure yielded 17 milligrams of purified UbBocK6 from 2L of culture.

To protect the six *N*ε-Lys amino groups and the *N*-terminal amine in UbBocK6 with Cbz groups we reacted the protein with 7 equivalents of *N*-(benzyloxycarbonyloxy)succinimide (Cbz-OSu) in basic DMSO²². After protection for 2 hours (Figure 3A) electrospray ionization mass spectrometry indicates that 7 or 8 Cbz groups are added to UbBocK6. The addition of 7 Cbz groups corresponds to the protection of all the free amino groups in UbBocK6. The additional Cbz group observed most likely corresponded to partial protection of the single histidine (his68) residue within ubiquitin. Since the protection is reversible, under subsequent deprotection conditions, over-protection is not a problem. We efficiently recovered the Boc and Cbz protected ubiquitin by precipitation and washing with cold ether and air-drying.

To reveal a single free N ε-Lys amino group at K6, as desired for isopeptide bond formation, we removed the Boc protecting group present in **1** with trifluorocetic acid (TFA) in water, leaving the Cbz protection intact (Figure 3A). The overall yield of this ligation-ready material (UbK6(Cbz₇₋₈)) from UbBocK6 was approximately 85 %.

### Example 2 - Preparation of a donor ubiquitin according to step (b) of the method of the invention

As mentioned above, when the other protein (in this case donor ubiquitin) also presents lysine side chains and the reaction involves linking a polypeptidic chain to another protein, it is advantageous to protect the lysine side chains present on the protein. This method is represented partly by the left hand side of Figure 1.

### Cloning of donor ubiquitin Ub1-76-thioester

The ubiquitin gene was PCR amplified from a plasmid containing Ub1-75 using the forward 5'-GGT GGT CAT ATG CAG ATC TTC GTC AAG ACG TTA ACC-3' primer and the reverse 5'-GGT GGT TGC TCT TCC GCA CCC GCC ACG CAG TCT TAA GAC CAG ATG-3' primer that introduced Ndel and Sapl restriction sites respectively. The reverse primer also inserted a codon for Gly 76. The PCR product was double digested with Ndel and Sapl restriction enzymes and ligated into similarly treated pTXBt vector (NEB) to create pTXB1-Ub1-76.

### Preparation of donor ubiquitin Ub1-76-thioester

ER2566 *E. coli.* cells (50 µL) (NEB) were transformed with pTXB1-Ub1-76 and recovered with S.O.B. medium (250 µL). The cells were incubated for 1 h at 37 °C and then LB medium (100 mL) containing ampicillin (100 µg mL⁻¹) was inoculated with the recovered cells (200 µl) and the culture was incubated overnight whilst shaking (230 rpm) at 37 °C. LB medium (2 L) containing ampicillin (100 µg mL⁻¹) was inoculated with the overnight culture (60 mL) and incubated whilst shaking (230 rpm) at 37 °C. At O.D.₆₀₀~ 0.4, the cells were transferred to a 25 °C incubator and after 30 min the cells were induced with IPTG (0.5 mM). After 5 h the cells were harvested and suspended in 60 ml lysis buffer (20 mM Na₂HPO₄ pH 7.2, 200 mM NaCl, 1 mM EDTA) and frozen. The thawed cells were lysed by sonication on ice and were clarified by centrifugation (39000 x g, 30 min). An empty XK 26/20 column was filled with chitin beads (20 mL) (NEB) and equilibrated with lysis buffer. At 4 °C the clarified lysate was loaded (flow rate:0.5 mL min⁻¹) onto the column using an ÄKTA FPLC system. The column was then washed with lysis buffer (~ 400 mL) and equilibrated with 60 mL of cleavage buffer (20 mM Na₂HPO₄ pH 6, 200 mM NaCl, 100 mM MESNa, 1 mM EDTA). The flow was then stopped and the column incubated for 66 h at 4 °C, to allow cleavage of the ubiquitin thioester (UbSR). Cleaved UbSR was eluted with elution buffer (20 mM Na₂HPO₄ pH 6, 200 mM NaCl, 1 mM EDTA). The fractions containing UbSR were determined by SDS-PAGE and were then pooled and concentrated to - 5 mL using an Amicon Ultra-15 centrifugal filter device (Millipore). The protein was then further purified by semi-preparative RP-HPLC employing a Phenomenex 250 mm x 10 mm, C18, 300 Å, 10 µm column. A gradient of 10 % buffer A to 75 % buffer B was applied at a flow rate of 5 mL min⁻¹ over 30 min (buffer A = 0.1 % TFA in H₂O, buffer B = 10 % buffer A in MeCN). Fractions containing ubiquitin thioester were verified by ESI-MS and were lyophilized.

### Global protection of Ub-MES thioester with Cbz-OSu

Lyophilized UbSR (10 mg, 1.15 famol) was dissolved in DMSO (833 µL) followed by the addition of DIEA (17 µL). Whilst stirring, Cbz-OSu (2.75 mg, 11.5 µmol) was added to the reaction. The reaction was stirred for 2 h at 25 °C and then transferred into cold ether (8.5 mL) and briefly vortexed. The precipitate was collected by centrifugation and the ether layer was discarded. The pellet was washed with ice-cold ether (8.5 mL) and air-\ dried.

### Results

A donor ubiquitin molecule was prepared biosynthetically as a C-terminal thioester (UbSR), by thiolysis of an intein fusion 11 with a purified yield of 6 mg per L of culture (Figure 3B, orange). To avoid the seven lysine residues or the N-terminal amine in UbSR participating in isopeptide bond formation we protected the free amines with 8 equivalents of Cbz-OSu using the conditions described for UbBocK6. The protection was complete within two hours, as judged by electrospray ionization mass-spectrometry (Figure 3B, blue), yielding UbSR(Cbz₈₋₉), which was isolated by ether precipitation.

### Example 3 - Specific isopeptide bond formation between donor and acceptor ubiquitin

UbK6(Cbz₇₋₈) (2.1 mg, 220 nmol) and UbSR(Cbz₇₋₈) (3.3 mg, 336 nmol) were dissolved in DMSO (90 µL). DIEA (4 µL), H-OSu (0.39 mg, 3.36 µmol) and AgNO₃ (57 µg, 336 nmol) were added. The reaction was incubated in the dark at 25 °C for 16 h. The crude mixture was precipitated with cold ether (1 mL) and washed with cold ether (1 mL) and air-dried. The proteins were dissolved in an ice-cold cocktail (5 mg/mL) consisting of 55 % TFA, 35 % DMS and 10 % TFMSA. After stirring at 0 °C for 90 min the proteins were precipitated with 10 volumes of cold ether followed by 0.5 % (vol.) pyridine. A heavy precipitate formed which was washed with cold ether, collected and dried. The dried precipitate was dissolved in buffer (100 mM Na₂HPO₄ pH 7.4, 8 M urea, 500 mM NaCl) at a protein concentration of approximately 0.5 mg/mL and dialyzed overnight against the same buffer (1 L) using a 3 kDa MWCO membrane (Spectrum Labs). The sample was then transferred to a fresh dialysis membrane and dialyzed overnight against folding buffer (20 mM Na₂HPO₄ pH 7.4, 100 mM NaCl). The protein was buffer exchanged into IEX buffer A (ammonium acetate pH 4.5) using an Amicon Ultra-15 3 kDa MWCO centrifugal filter device (Millipore). The sample was filtered (0.45 µM) and loaded onto a pre-equilibrated MonoS 5/50 GL column (GE Life Sciences) at a flow rate of 0.5 mL/min using an ÄKT A FPLC system. The flow was increased to 2 mL/min and a gradient running to 60 % IEX buffer B (ammonium acetate pH 4.5, 1 M NaCl) over 10 minutes was applied. Fractions (0.5 mL) were collected and those containing K6-linked diubiquitin were determined by SDS-PAGE. The fractions were pooled and exchanged into IEX buffer A again using a centrifugal filter device. The sample was reapplied to the equilibrated MonoS column and at a flow rate of 2 mL/min a gradient running to 60 % buffer B over 45 minutes was applied. Fractions were collected in 1 mL volumes and those containing pure K6-linked diubiquitin were pooled and concentrated to 1 mg/mL (200 - 300 µg, 5 - 8 % yield). K6-linked diubiquitin was then dialyzed overnight against storage buffer (10 mM Tris-HCl pH 7.6) using a Dispo Biodialyzer 5 kDa MWCO (The Nest Group Inc.). The K6-linked diubiquitin samples were frozen at -20 °C for storage. Preparation of K29-linked diubiquitin was carried out as described for K6-linked diubiquitin except UbK29(Cbz7-8) was used in the isopeptide bond forming reaction.

### Results

Thioesters can be activated and converted *in situ* to *N-*hydroxysuccinimidyl esters in the presence of Ag(l), allowing selective acylation with amines ^{23 24}. We realized that this chemistry might be applied to the formation of a specific isopeptide bond between UbK6(Cbz₇₋₈) and UbSR(Cbz₈₋₉). We mixed the donor ubiquitin thioester (UbK6(Cbz₇₋₈)) with UbSR(Cbz₈₋₉) at a molar ratio of 1:1.5 in DMSO, in the presence of DIEA, silver nitrate (AgNO₃, 3.75 mM) and N-hydroxysuccinimide (H-OSu, 37.5 mM) (Figure 4A). After 16 hours at room temperature the crude ligation reaction was precipitated and washed with cold ether. Analysis by SDS-PAGE reveals that approximately 30% of the protein band containing ubiquitin monomers (UbK6(Cbz₇₋₈) & UbSR(Cbz₈₋₉) ) has been converted to a higher molecular weight species, corresponding to the Cbz protected ligation product.

To remove the Cbz groups we used a cleavage cocktail consisting of 1:3:6 trifluoromethanesulfonic acid (TFMSA):trifluoroacetic acid (TFA):dimethylsulfide (DMS) at 0 °C for 1 hour²⁵. The deprotected ubiquitin chain was precipitated, washed and resuspended in PBS buffer containing 8M Urea to form an unfolded ubiquitin chain. Since ubiquitin folds reversibly and enzymatically synthesized K48- and K68-linked ubiquitin chains are purified and refolded in vitro from denatured material²⁶ it seemed reasonable that we could refold the atypical ubiquitin chains. We therefore dialyzed the protein into PBS buffer lacking urea to slowly re-nature the linked diubiquitin. Subsequent cation exchange allowed removal of residual monoubiquitin species, resulting in highly purified K6-linked diubiquitin (Figure 4B, Figure 5A & B).

To synthesize K29-linked ubiquitin we simply repeated the procedure described, except that we used *UbTAG*29-*His*₆, in which the amber codon is at position 29, in place of *UbTAG6-His₆.* For the preparation of UbBocK29 a yield of 8 mg from a 2 L culture was obtained.

### Example 4- Characterization & structure of K6-linked diubiquitin

To demonstrate that the purified K6- and K29-linked diubiquitin synthesized using the method according to the invention was linked via an isopeptide bond at the genetically directed site, and fully deprotected we used ESI-MS, and MS/MS sequencing.

Protein total mass was determined on an LCT time-of - flight mass spectrometer with electrospray ionization (Micromass). Samples were dissolved in 1:1 acetonitrile/H₂O containing 1 % formic acid. In the case of Cbz protected peptides, samples were dissolved in 4:3:3 acetic acid/acetonitrile/H₂O. Samples were injected at 10 µl min⁻¹ and calibration was performed in positive ion mode using horse heart myoglobin. 30 scans were averaged and molecular masses obtained by maximum entropy deconvolution with MassLynx version 4.1 (Micromass). Theoretical masses of wild-type proteins were calculated using Protparam (http://us.expasy.org/tools/protparam.html), and theoretical masses for unnatural amino acid containing proteins were adjusted manually.

For MS/MS analyses samples were digested with trypsin overnight at 37°C. Samples were then desalted and analyzed by LC-MS/MS with a LTQ Orbitrap Mass Spectrometer (Thermo Scientific). Target peptides were fragmented by collision-induced dissociation.

ESI-MS reveals a single mass-peak for the purified proteins, which corresponds to an isopeptide linked, quantitatively deprotected diubiquitin. (K6-linked observed mass = 17113 Da, K29-linked observed mass = 17111 Da, calculated mass = 17112 Da for diubiquitin; Figures 4C & 4D). To demonstrate that the isopeptide bond is formed at the K6 and K29 positions we performed MS/MS sequencing on the linked diubiquitins, which verifies the specific formation of the K6 and K29 isopeptide linkages (Figures 4E & 4F), with a fidelity of greater than 95%.

We were able to solve the structure of K6-linked diubiquitin by X-ray crystallography.

K6 diubiquitin crystallised with cubic morphology at a protein concentration of 1-2 mg mL⁻¹ in hanging drops equilibrated against 19-20 % PEG 3350, 0.2 M zinc acetate. Before freezing in a nitrogen cryostream, the crystals were soaked in mother liquor supplemented with 15% PEG 400. The largest crystals (30 µm) diffracted to 3 Å at the European Synchrotron Radiation Facility (ESRF) on beamline ID14-2. Initial phases were obtained by molecular replacement using one ubiquitin moiety from the deposited coordinates of a K63 diubiquitin structure (pdb-id 2JF5, ⁷). Structure refinement was carried out with PHENIX ⁴⁵ and model building was carried out within COOT ⁴⁵. In final rounds of refinement, TLS temperature factor refinement was performed. Geometric weight optimization in PHENIX resulted in a model with the lowest R/Rfree factors. Data collection and refinement statistics are shown in Table 1.

Crystals grown from 20% PEG 3350 and 200 mM ZnAc formed in a cubic space group (P4₃32) and diffracted to 3.0 Å resolution. The structure of K6-linked diubiquitin was solved by molecular replacement and subsequently refined (the statistics for refinement are shown in Table 1), revealing one K6-linked diubiquitin molecule in the asymmetric unit. Each ubiquitin adopts a native conformation confirming that the success of the refolding step in GOPAL. While ESI and MS/MS demonstrated the formation of the K6 isopeptide bond, the flexible isopeptide linkage is not fully resolved in the electron density maps, and Gly76 does not display discernible electron density. This has previously been observed in polyubiquitin structures due to the high flexible of the linkage.

The structure of K6-linked diubiquitin reveals that it adopts an asymmetric compact conformation distinct from previously described ubiquitin chain structures (Figure 6a & 6e). The proximal ubiquitin moiety (resulting from the acceptor ubiquitin and containing Lys6 that contributes to the isopepeptide bond), binds via a hydrophobic surface surrounding Ile44 and Val70 to the distal ubiquitin (resulting from the donor ubiquitin that contributes its C-terminus to the isopeptide bond). A second, distinct hydrophobic patch, containing Leu71, Ile36 and Leu8 (hereafter refereed to as the Ile36 patch), acts as the hydrophobic counterpart on the distal ubiquitin molecule. The extended asymmetric interface results in a compact diubiquitin molecule (Figure 6b). Additional interface residues are Arg42 in the proximal ubiquitin, which contributes to the interaction surface, and Thr9 in the proximal ubiquitin that forms a hydrogen bond with Gln49 in the distal ubiquitin (Figure 6c).

The K6 ubiquitin-ubiquitin interaction interface displays several previously undescribed features. The Ile44-patch is a common ubiquitin interaction interface, observed in the majority of interactions with ubiquitin binding domains and deubiquitinases ^{8,27}. In this interaction patch Val70 and Leu8 flank the central Ile44 residue, providing an extended hydrophobic interface (Figure 6d). In K6-linked diubiquitin, the Ile44-patch is smaller, since Leu8 undergoes a conformational change to participate in a distinct, almost perpendicular interface. This novel interface also contains Leu71 and Ile36, which together form an apolar surface of ~480 Å², which we term the Ile36 patch, and which interacts with the Ile44/Val70 residues of a proximal molecule. This asymmetric interaction then leaves the Ile44/Val70 patch of the distal molecule exposed, and available for chain extension via K6 or for binding to ubiquitin binding domains (Figure 6b).

We were able to build a model hexamer from the asymmetric K6 dimer by iteratively superimposing (in Coot ²⁸) the proximal ubiquitin moiety of one dimer onto the distal ubiquitin of a second dimer, to generate a new distal moiety (Figure 6f). No steric clashes are observed for any ubiquitin molecules in the hexamer. Instead, novel, smaller interactions (-83 Å²) between n+2 and n-2 ubiquitin molecules are facilitated (i.e. between molecules A-C, and C-E, in Figure 5g), as revealed by analysis in the PISA server. More importantly, the generated K6-hexamer model folds into a five-fold symmetric helical filament (Figure 6g). Six molecules form two turns of the helix, and molecule A and molecule F are in equivalent relative orientations, translated by 62 Å along the helical axis. Since this symmetry is not generated through crystallographic or lattice contacts, our model suggests that K6-linked ubiquitin chains may form symmetric biological molecules.

### Example 5 - Profiling deubiquitinases on K6- 8. K29-linked ubiquitin DUB generation and deubiquitinase assays

The pRSET-UCHL3 plasmid was a generous gift from Keith D. Wilkinson, and was used prepare pure UCH-L3 as previously described ⁴³. An OTUB 1 expression vector was kindly provided by Benedikt Kessler (Oxford), and the protein was purified according to ⁴⁴. USP21 was cloned from a plasmid kindly provided by Sylvie Urbe (Liverpool). Purification of remaining deubiquitinases is described in ⁷. USP2, USP15, and BAP1 were purchased from ENZO LifeSciences or Boston Biochem. DUB assays were carried out as previously described ⁷.

### Results

Deubiquitinases may be endowed with preference for particular chain linkages ²⁷. However, since most ubiquitin chain types have not been synthesized, deubiquitinase specificity profiling is incomplete. A mixture of K6 linked and K29 linked diubiquitin molecules, in which other lysine residues in the distal and proximal molecules were mutated to arginine, were examined for cleavage with hOtu1, an OTU family deubiquitinase, and two JAMM deubiquitinase complexes ^{29,30}. These deubiquitinases were found not to cleave either chain type, but these experiments are problematic because it is unclear whether the mutated ubiquitin chains reflect the properties of the native chain. Deubiquitinases cover a large surface area in particular on the distal ubiquitin molecule²⁷ and mutation of surface Lys residues to Arg may interfere with deubiquitinase binding. We analyzed twelve deubiquitinases representing approximately 10 % of known human deubiquitnases and covering four deubiquitinase families - ubiquitin C-terminal hydrolases (UCH), ubiquitin specific proteases (USP), Ovarian Tumor (OTU) deubiquitinases, and JAMM/MPN+ deubiquitinases - for their ability to cleave K6- and K29-linked diubiquitin in *in vitro* deubiquitinase assays ⁷. In control experiments we used the same assay to cleave enzymatically assembled K63-linked diubiquitin. UCH enzymes are highly efficient in cleaving small unstructured peptides from the C-terminus of ubiquitin (such as the His-tag, Figure 2), or hydrolyze ubiquitin from unstructured proteins/loops, but do not hydrolyze native K63-, K48- and linear ubiquitin chains ^{27,31}. UCH-L3 and BAP1 failed to cleave K6- and K29-linked diubiquitin molecules (Figure 7), demonstrating that ubiquitin polymers with these linkages are unlikely substrates for these UCH enzymes.

In contrast to UCH enzymes, USP deubiquitinases are highly active in cleaving ubiquitin polymers, but often without obvious linkage specificity ⁷. An exception is the tumor suppressor CYLD, whose USP domain prefers K63-linkages over K48-linkages³². When tested with K6-linked diubiquitin, four USP domains (USP2, USP5, USP 15, USP21) disassembled this chain type as efficiently as K63-linked chains (Figure 7). While the K29-linked ubiquitin was rapidly cleaved by USP5 it displayed appreciably higher resistance to hydrolysis by USP2, USP15 and USP21 than K6- and K63-linked ubiquitin. USP5 is a promiscuous DUB, which recognizes the C-terminus of a free ubiquitin chain specifically³³, and functions to replenish the ubiquitin pool by hydrolyzing unattached ubiquitin chains²⁷. In contrast, CYLD showed a markedly decreased activity against the K6-and K29-linkage with respect to its preferred K63-linkage. This demonstrates that CYLD's prefers K63-linkages over K6- and K29-linkages as well as K48-linkages.(Figure 7).

OTU domain deubiquitinases hydrolyze polyubiquitin chains, yet some members display remarkable selectivity between K48- and K63-linkages. A20 and OTUB1 are specific for K48-linked chains, and do not hydrolyze K63-linked or linear chains ^{7,29}, while TRABID is K63-linkage specific ^{7,17}. The OTU domains tested (A20, TRABID, Cezanne and OTUB1) did not cleave K6-linked diubiquitin, and A20, Cezanne and OTUB1 also did not cleave K29-linkages (Figure 7) at concentrations where they hydrolyze their preferred chains type completely, though at higher enzyme concentration an appreciable activity against all linkages was observed. This extends the previously reported specificity with these enzymes to specificity with respect to K6- and K29-linkages. Interestingly, TRABID cleaves K29-linkages with similar if not higher activity compared to K63-linkages, indicating that this enzyme has a dual specificity: preferring K29 and K63 linkages to K48 and K6 linkages. Given that OTU domains appear to be intrinsically specific for a subset of linkages, it is possible that other family members can cleave other atypical ubiquitin chains specifically. Extensions of our approach should allow us to discover these activities.

JAMM domain deubiquitinases have also been reported to be K63-linkage specific ^{30,34}, and the molecular basis for recognizing the K63-linkage was unveiled in the recent crystal structure of AMSH in complex with K63-linked diubiquitin ³⁵. AMSH binds to the extended K63-linked diubiquitin molecule and makes specific contacts with residues surrounding Lys63. We find that AMSH is inactive against K6- and K29-linked diubiquitin, while cleaving K63-linked diubiquitin under identical reaction conditions with high activity.

**Table 1 - Data collection and refinement statistics for K6-linked diubiquitin. Values between brackets are for the highest resolution shell.**

| | K6-linked diubiquitin |
|---|---|
| ***Data collection statistics*** | |
| Beamline | ID14-2 |
| Wavelength (Å) | |
| Space Group | P4₃32 |
| Unit Cell (Å) | *a,b,c* = 105.02, α,β,γ=90 |
| Resolution (Å) | 52.41-3.00 (3.16-3.00) |
| Observed reflections | 20871 (2995) |
| Unique reflections | 4247 (591) |
| Redundancy | 4.9 (5.1) |
| Completeness (%) | 99.0 (98.6) |
| Rmerge | 0.070 (0.582) |
| <\|/σ\|> | 13.5 (2.6) |
| | |

| ***Refinement statistics*** | |
|---|---|
| Reflections in test set | 503 |
| *R*_{cryst} | 0.213 |
| *R*_{free} | 0.249 |
| Number of groups | |
| Protein residues | 148 |
| Zinc ions | 7 |
| Wilson B (Å²) | 102.9 |
| <*B*> protein (Å²) | 84.9 |
| <*B*> water (Å²) | 104.2 |
| | |
| RMSD from ideal geometry | |
| Bond length (Å) | 0.004 |
| Bond angles (°) | 0.771 |

### Example 6: Engineered ubiquitin synthesis reveals Lys29-isopeptide specificity of an OTU deubiquitinase

Ubiquitination is a reversible post-translational modification that regulates a myriad of eukaryotic functions. Our ability to study the effects of ubiquitination is often limited by the inaccessibility of homogeneously ubiquitinated proteins. In particular, elucidating the roles of the so-called 'atypical' ubiquitin chains (chains other than Lys48- or Lys63-linked ubiquitin), which account for a large fraction of ubiquitin polymers, is challenging because the enzymes for their biosynthesis are unknown. Here we combine genetic code expansion, intein chemistry and chemoselective ligations to synthesize `atypical' ubiquitin chains. We solve the crystal structure of Lys6-linked diubiquitin, which is distinct from that of structurally characterized ubiquitin chains, providing a molecular basis for the different biological functions this linkage may regulate. Moreover, we profile a panel containing 10% of the known human deubiquitinases on Lys6- and Lys29-linked ubiquitin and discover that TRABID cleaves the Lys29 linkage 40-fold more efficiently than the Lys63 linkage.

Ubiquitination is a reversible post-translational modification in which a specific lysine residue in an acceptor protein forms an isopeptide bond with the C terminus of the ubiquitin donor. Although the role of ubiquitination in regulating protein stability via proteasomal targeting is well established, it is emerging that ubiquitin is involved in almost every aspect of biology, including cell signaling, intracellular trafficking and the response to DNA damage^{1,2}. Ubiquitin forms covalent chains through each of its seven lysine residues (Lys6, Lys11, Lys27, Lys29, Lys33, Lys48 or Lys63) or its N terminus, and it is proposed that the distinct functions mediated by ubiquitin in diverse biological processes may be encoded in the distinct properties of the different ubiquitin chains^{2,3}.

Although proteomic studies reveal that all chain types are present *in vivo*^{4,5}, we know the most about Lys48- and Lys63-linked chains, which are important in proteasomal degradation, and cell signaling, respectively^{1,6}. In contrast, very little is known about the other so-called 'atypical' linkages, though they account for more than half of the ubiquitin linkages found in the model organism *Saccharomyces cerevisiae⁵.* A central challenge in studying the roles of specific ubiquitin chains is to synthesize homogeneous chains bearing defined linkages. Indeed, access to Lys48- and Lys63-linked ubiquitin, via identification of the cellular machinery (E1, specific E2 and E3 enzymes) that allows their specific biosynthesis *in vitro,* has allowed the characterization of their biological roles². The structures of these chains have revealed distinct features, which may provide the molecular basis by which different chains are recognized by specific deubiquitinases or ubiquitin-binding domains^{7,8}. Moreover, access to homogeneous chains has facilitated the generation of linkage-specific antibodies^{9,10}, allowing the roles of specific chain types to be probed *in vivo.* Overall, the capacity to synthesize Lys48- and Lys63-linked ubiquitin chains has greatly accelerated our understanding of how these chain types are specifically recognized and regulated to mediate distinct biological processes, and we recently reported a biosynthesis of Lys11-linked chains, which should give further insights into this linkage¹¹.

Unfortunately, the specific enzymes required to synthesize the atypical ubiquitin linkages are simply unknown, severely limiting our ability to study the structure and function of these chain types. In principle, chemical ligation approaches might be used to synthesize ubiquitin chains. Indeed, a lysine derivative containing a ligation auxiliary has been used to generate a native isopeptide bond between a histone and ubiquitin without mutation of adjacent residues^{12,13}. However, this strategy involves multiple rounds of native chemical ligation and selective deprotection, yields small amounts of material and is best suited for ligation sites close to the termini of a target protein. Other ligation auxiliaries that yield an isopeptide linkage have been reported ^{14,15}, but their utility for linking entire proteins via a native isopeptide linkage has not been demonstrated. Moreover, these auxiliaries generally create glycine-to-alanine or glycine-to-cysteine mutations at the C terminus of the donor ubiquitin via nontraceless ligation reactions^{14,16}. Although these mutations may not be important for some studies, they are known to abrogate the action of deubiquitinases¹⁷ and may alter the structure and dynamics of the linkage in unpredictable ways.

Here we report a new approach for the synthesis of homogeneously linked ubiquitin chains that allows the synthesis of specific chains in the absence of the cellular machinery for their synthesis. Our approach uses a powerful combination of genetic code expansion and chemoselective protein chemistry, which we term GOPAL (genetically encoded orthogonal protection and activated ligation). We demonstrate the generality of GOPAL by preparing Lys6- and Lys29-linked ubiquitin chains, which constitute 10% and 3% of ubiquitin linkages in yeast, respectively⁵. We solve the crystal structure of a Lys6-linked diubiquitin molecule synthesized by GOPAL and reveal that the molecule adopts a compact conformation distinct from the structures of Lys48- or Lys63-linked chains. Moreover, access to Lys6- and Lys29-linked diubiquitin allows us to profile a panel of 11 deubiquitinases (constituting approximately 10% of human deubiquitinases) for their ability to cleave these linkages. Using a quantitative deubiquitinase assay, we reveal that the ovarian tumor (OTU) family deubiquitinase TRABID, which has previously been shown to have high specificity for Lys63-linked ubiquitin over Lys48-linked ubiquitin¹⁸, has a 40-fold specificity for the Lys29 linkage over the Lys63 linkage.

### RESULTS

### A strategy for specific isopeptide bond formation

The specific formation of an isopeptide bond between the C terminus of one ubiquitin (donor) and the amine of a specific lysine residue in another ubiquitin (acceptor) requires these functional groups to be differentiated from the other carboxylic acids and amines in both ubiquitin molecules (**Figure 8**). We realized that specific isopeptide bond formation might be achieved in a series of steps, by (i) protecting all lysines and the N terminus on the donor ubiquitin and all the lysines but one and the N terminus on the acceptor ubiquitin, giving a single free amine on the acceptor ubiquitin, (ii) specifically activating the C terminus of the donor ubiquitin as a thioester, (iii) forming a specific isopeptide bond between donor and acceptor ubiquitins by selectively coupling the free amine and the thioester, (iv) removing all the protecting groups to reveal the ubiquitin conjugate and (v) refolding the ubiquitin chain. To exemplify the generality of this approach, we aimed to make ubiquitin dimers linked through a specific isopeptide bond between the C terminus of the donor ubiquitin and an ε-amino group of a lysine residue on the acceptor ubiquitin (either Lys6 or Lys29). As each ubiquitin contains seven lysines and an N-terminal amino group, this system provides a stringent test of the specificity of our approach.

### Generation of ubiquitin ligation precursors

Protecting all but one lysine residue in a protein requires the differentiation of chemically identical amino acid side chains. Indeed, although several reactions are known that are specific for one type of residue, it has been much more challenging to site-specifically modify proteins on one residue in the presence of many chemically identical residues. We realized that this problem might be solved by genetically encoding the site-specific incorporation of a protected version of lysine. Subsequent protection of all other amines in the protein with a chemically orthogonal protecting group and specific removal of the genetically encoded protecting group would yield a lysine with a free amine at the site where the protected lysine was genetically encoded (**Figure 8**).

To site-specifically install a protected lysine at position 6 of ubiquitin, we took advantage of the *Methanosarcina barkeri MS* pyrrolysine tRNA synthetase (*Mb*PylRS) and its cognate amber suppressor tRNA (*Mb*tRNA_{CUA})¹⁹, which directs the efficient incorporation of *N*ε-*(t*-butyloxycarbony1*)*-L-lysine (**1**) into recombinant proteins in response to the amber codon in *Escherichia coli*²⁰⁻²². We created a ubiquitin expression construct in which the ubiquitin gene contains a TAG codon in place of the lysine codon at position 6 and is flanked by a 3' His₆ tag coding sequence (*UbTAG6-His₆*). We produced UbBocLys6-His₆ (ubiquitin-His₆ containing **1** at position 6) by expressing *UbTAG6-His₆* in cells containing the *Mb*PylRS and *Mb*tRNA_{CUA} pair and **1** (2 mM). UbBocLys6-His₆ production was strictly dependent on the addition of **1** (data not shown). UbBocLys6-His₆ was purified by Ni-NTA chromatography, and the His₆ tag was removed by treatment with ubiquitin C-terminal hydrolase-L3 (UCH-L3) to give UbBocLys6 (**Supplementary** **Fig. 1**). The untagged UbBocLys6 was then further purified by size-exclusion chromatography, desalted and lyophilized. The purified material was characterized by ESI-MS (**Fig**. **9a**). This procedure yielded 17 mg of purified UbBocLys6 from 2 liters of culture.

To protect the six *Nε-*Lys amino groups and the N-terminal amine in UbBocLys6 with Cbz groups, we reacted the protein with seven equivalents of *N-*(benzyloxycarbonyloxy)succinimide (Cbz-OSu) in basic DMSO²³. After 2 h of protection (**Fig**. **9a**), ESI-MS was performed and indicated that seven or eight Cbz groups are added to UbBocLys6. The addition of seven Cbz groups corresponds to the protection of all the free amino groups in UbBocLys⁶. The additional Cbz group that was observed most likely corresponded to partial protection of the single histidine (His68) residue within ubiquitin. As the protection is reversible under subsequent deprotection conditions, overprotection is not a problem. We efficiently recovered the Boc- and Cbz-protected ubiquitin by precipitation and washing with cold ether and air drying. To reveal a single free *Nε*-Lys amino group at Lys6, as desired for isopeptide bond formation, we removed the Boc protecting group present in **1** with trifluoroacetic acid (TFA) in water, leaving the Cbz protection intact (**Fig. 9a**). The overall yield of this ligation-ready material (UbLys6(Cbz₇₋₈)) from UbBocLys6 was approximately 85%.

A donor ubiquitin molecule was prepared biosynthetically as a C-terminal thioester (UbSR), by thiolysis of an intein fusion¹² with a purified yield of 6 mg per liter of culture (**Fig. 9b****,**, orange, and **Supplementary** **Fig. 2**). To avoid the participation of the seven lysine residues or the N-terminal amine in UbSR in isopeptide bond formation, we protected the free amines with eight equivalents of Cbz-OSu using the conditions described for UbBocLys6. The protection was complete within 2 h, as judged by ESI-MS (**Fig**. **9b**, blue), yielding UbSR(Cbz₈₋₉), which was isolated by ether precipitation.

### Specific, enzyme-independent, isopeptide bond formation

Thioesters can be activated and converted *in situ* to *N*-hydroxysuccinimidyl esters in the presence of Ag(l), allowing selective acylation with amines^{24,25}. We realized that this chemistry might be applied to the formation of a specific isopeptide bond between UbLys6(Cbz₇₋₈) and UbSR(Cbz₈₋₉). We mixed the donor ubiquitin thioester (UbLys6(Cbz₇₋₈)) with UbSR(Cbz₈₋₉) at a molar ratio of 1:1.5 in DMSO, in the presence of *N*,*N*-diisopropylethylamine (DIEA), silver nitrate (AgNO₃, 3.75 mM) and *N*-hydroxysuccinimide (H-OSu, 37.5 mM) (**Supplementary** **Fig. 2**). After 16 h at room temperature, the crude ligation reaction was precipitated and washed with cold ether. Analysis by SDS-PAGE revealed that approximately 30% of the protein band containing ubiquitin monomers (UbLys6(Cbz₇₋₈) & UbSR(Cbz₈₋₉)) had been converted to a species of higher molecular weight, corresponding to the Cbz-protected ligation product (**Supplementary** **Fig. 2**).

To remove the Cbz groups, we used a cleavage cocktail consisting of 1:3:6 trifluoromethanesulfonic acid (TFMSA)/trifluoroacetic acid (TFA)/DMS at 0 °C for 1 h²⁶. The deprotected ubiquitin chain was precipitated, washed and resuspended in PBS buffer containing 8 M urea to form an unfolded ubiquitin chain. Because ubiquitin folds reversibly, and enzymatically synthesized Lys48- and Lys68-linked ubiquitin chains are purified and refolded *in vitro* from denatured material²⁷, it seemed reasonable that we could refold the atypical ubiquitin chains. We therefore dialyzed the protein into PBS buffer lacking urea to slowly renature the linked diubiquitin. Subsequent cation exchange allowed removal of residual monoubiquitin species, resulting in highly purified Lys6-linked diubiquitin (**Supplementary** **Fig. 2**).

To synthesize Lys29-linked ubiquitin, we simply repeated the procedure described, except that we used *UbTAG29-His₆-in* which the amber codon is at position 29-in place of *UbTAG6-His₆,* For the preparation of UbBocLys29, a yield of 8 mg from a 2-liter culture was obtained. The subsequent steps in generating Lys29-linked diubiquitin proceeded with efficiency comparable to that of the steps described in detail for the preparation of Lys6-linked diubiquitin.

### Characterization of Lys6- and Lys29-linked diubiquitin

To demonstrate that the purified Lys6- and Lys29-linked diubiquitin synthesized using GOPAL was linked via an isopeptide bond at the genetically directed site, as well as fully deprotected, we used ESI-MS and MS/MS sequencing. ESI-MS revealed a single mass peak for the purified proteins, which corresponded to an isopeptide-linked, quantitatively deprotected diubiquitin (Lys6-linked diubiquitin, observed mass = 17,113 Da; Lys29-linked diubiquitin, observed mass = 17,111 Da; diubiquitin calculated mass = 17,112 Da; **Fig. 9c****,d**). To demonstrate that the isopeptide bond is formed at the Lys6 and Lys29 positions, we performed MS/MS sequencing on the linked diubiquitin molecules, a process that verifies the specific formation of the Lys6 and Lys29 isopeptide linkages (**Fig. 9e****,f**), with a fidelity of greater than 95%.

### Crystal structure of Lys6-linked diubiquitin

We were able to determine a structure for Lys6-linked diubiquitin by X-ray crystallography. Crystals grown from 20% PEG 3350 and 200 mM ZnAc formed in a cubic space group (*P*4₃32) and diffracted to 3.0-Å resolution. The structure of Lys6-linked diubiquitin was solved by molecular replacement and subsequently refined (for statistics see **Supplementary Table 1**), revealing one Lys6-linked diubiquitin molecule in the asymmetric unit. Each ubiquitin adopts a native conformation, confirming the success of the refolding step. Although ESI-MS and MS/MS demonstrated the formation of the Lys6 isopeptide bond, the flexible isopeptide linkage is not fully resolved in the electron density maps, and Gly76 does not show discernible electron density. This has previously been observed to occur in polyubiquitin structures because of the high flexibility of the linkage (**Supplementary** **Fig. 3a**).

The crystal structure of Lys6-linked diubiquitin reveals an asymmetric compact conformation distinct from previously described ubiquitin chain structures (**Fig. 10**). The proximal ubiquitin moiety (arising from the acceptor ubiquitin and containing Lys6 that contributes to the isopeptide bond) binds via a hydrophobic surface surrounding Ile44 and Val70 to the distal ubiquitin (arising from the donor ubiquitin that contributes its C terminus to the isopeptide bond). A second, distinct hydrophobic patch, containing Leu71, Ile36 and Leu8 (hereafter referred to as the Ile36 patch), acts as the hydrophobic counterpart on the distal ubiquitin molecule. The extended asymmetric interface results in a compact diubiquitin molecule (**Fig. 10b****,c**). Additional interface residues are Arg42 and Gln49 in the proximal ubiquitin and Gln40 and Thr9 in the proximal ubiquitin. A hydrogen bond between Thr9 and Gln49 is formed (**Fig. 10d**).

The Lys6 ubiquitin-ubiquitin interaction interface has several previously undescribed features. The hydrophobic patch surrounding Ile44 is a common ubiquitin interaction interface, observed in the majority of interactions with ubiquitin binding domains and deubiquitinases^{8,28}. In this interaction patch, Val70 and Leu8 flank the central Ile44 residue, providing an extended hydrophobic interface (**Fig. 10e**). In Lys6-linked diubiquitin, the Ile44-patch is smaller, as Leu8 undergoes a conformational change to participate in a distinct, almost perpendicular interface. This new interface also contains Leu71 and Ile36, which together form an apolar surface of -480 Å², termed the Ile36 patch. This interacts with the Ile44 and Val70 residues of a proximal molecule. This asymmetric interaction then leaves the Ile44 and Val70 patch of the distal molecule exposed and available for binding to ubiquitin binding domains (**Fig. 10b,10d**). In addition, the diubiquitin model allows chain extension via the asymmetric interface. Iterative modeling of a longer Lys6-linked ubiquitin chain on the basis of the diubiquitin structure suggests formation of a helical filament for an extended Lys6-linked polymer (**Supplementary** **Fig. 3**).

The structure of Lys6-linked diubiquitin is distinct from previously observed diubiquitin structures (**Fig. 10f****)** and is also different from a recently suggested computational model, which assumed a symmetric interaction involving the Ile44 surface on both sides of the interface²⁹. However, because of the dynamic nature of polyubiquitin chains in solutions, it is possible that distinct interfaces and/or less compact conformations can be adopted. Future studies with nuclear magnetic resonance and at the single-molecule level will be required to further understand the structural features of Lys6-linked polyubiquitin.

### Profiling deubiquitinases on Lys6- and Lys29-linked ubiquitin

Deubiquitinases may be endowed with preference for particular chain linkages²⁸. However, as most ubiquitin chain types have not been synthesized, deubiquitinase specificity profiling is incomplete. In previous work, a mixture of Lys6- and Lys29-linked diubiquitin molecules, in which additional lysine residues in the distal and proximal molecules were mutated to arginine, were examined for cleavage with hOtu1, an OTU family deubiquitinase, and two JAMM/MPN+ deubiquitinase complexes ^{30,31}. These deubiquitinases were found not to cleave either chain type, but these experiments are problematic because it is unclear whether the mutated ubiquitin chains reflect the properties of the native chain. Most deubiquitinases interact extensively with the distal ubiquitin molecule²⁸, and mutation of surface lysine residues to arginines may interfere with deubiquitinase binding.

We analyzed 11 deubiquitinases, representing approximately 10% of known human deubiquitinases and covering four deubiquitinase families-ubiquitin C-terminal hydrolases (UCH), ubiquitin-specific proteases (USP), OTU deubiquitinases and JAMM/MPN+ deubiquitinases-for their ability to cleave Lys6- and Lys29-linked diubiquitin in *in vitro* deubiquitinase assays⁷. In control experiments, we used the same assay to cleave enzymatically assembled Lys63-linked diubiquitin. To discover enzymes that cleave Lys6- or Lys29-linked diubiquitin in preference to Lys63-, Lys48-or Lys11-linked or linear chains, we performed our assays under conditions in which the enzyme efficiently cleaves one of these previously analyzed chain types. Any enzyme that does not cleave Lys6- or Lys29-linked diubiquitin under these conditions is unlikely to have these linkages within its repertoire of substrates. However, if the Lys6-or Lys29-linked diubiquitin is an efficient substrate under these conditions, then the substrate specificity of the enzyme merits further investigation.

UCH enzymes are highly efficient in cleaving small, unstructured peptides from the C terminus of ubiquitin (such as the His tag, **Supplementary** **Fig. 1**) or in hydrolyzing ubiquitin from unstructured proteins or loops, but they do not hydrolyze native Lys63-, Lys48- or Lys11-linked or linear ubiquitin chains^{11,28,32}. UCH-L3 and BAP1 failed to cleave Lys6- and Lys29-linked diubiquitin molecules **(****Fig. 11** and **Supplementary** **Fig. 4**), confirming that ubiquitin polymers with these linkages are unlikely substrates for these UCH enzymes.

In contrast to UCH enzymes, USP deubiquitinases are highly active in cleaving ubiquitin polymers, though often without obvious linkage specificity⁷. USP2 cleaved Lys6- and Lys63-linked diubiquitin more rapidly than Lys29-linked diubiquitin (**Fig. 11** and **Supplementary** **Figs. 5** and **6**). USP5 is a promiscuous deubiquitinase, which specifically recognizes the C terminus of a free ubiquitin chain³³ and functions to replenish the ubiquitin pool by hydrolyzing unattached ubiquitin chains²⁸. USP5 rapidly cleaves all chains types tested, and we observe little discrimination in our assays with this enzyme (**Fig. 11** and **Supplementary** **Figs. 5** and **6**). Similarly, USP21 rapidly cleaves all chain types tested (**Fig. 11** and **Supplementary** **Figs. 5** and **6**). In contrast, the tumor suppressor CYLD, whose USP domain is known to prefer Lys63 linkages over Lys48 linkages³⁴, shows little or no activity against the Lys6 and Lys29 linkage but efficiently cleaves its preferred Lys63 linkage. This demonstrates that CYLD prefers Lys63-linked ubiquitin over Lys6 and Lys29 linkages (**Fig. 11**).

JAMM domain deubiquitinases have also been reported to be Lys63-linkage Specific^{31,35}, and the molecular basis for recognizing the Lys63 linkage was unveiled in the recent crystal structure of AMSH-LP in complex with Lys63-linked diubiquitin³⁶. AMSH binds to the extended Lys63-linked diubiquitin molecule and makes specific contacts with residues surrounding Lys63. We find that AMSH is inactive against Lys6-and Lys29-linked diubiquitin, although it cleaves Lys63-linked diubiquitin under identical reaction conditions with high activity.

OTU domain deubiquitinases hydrolyze polyubiquitin chains, yet some members show remarkable selectivity between Lys48 and Lys63 linkages. A20 and OTUB1 are specific for Lys48-linked chains and do not hydrolyze Lys63-linked or linear chains^{7,30}, whereas TRABID has a preference for Lys63 linkages^{7,18}. Similarly, Cezanne has a preference for Lys11 linkages over Lys48 and Lys63 linkages¹¹. The OTU domains tested (A20, TRABID, Cezanne and OTUB1) did not cleave Lys6-linked diubiquitin, and A20, Cezanne and OTUB1 also did not cleave Lys29 linkages (Fig. 11) under the assay conditions in which these enzymes hydrolyze their preferred chain type completely. This extends the previously reported specificity of these enzymes with respect to Lys6 and Lys29 linkages. Our qualitative measurements (**Fig. 11**) indicated that TRABID cleaves Lys29 linkages more rapidly than Lys63 linkages.

### Kinetics of ubiquitin chain hydrolysis by TRABID

To investigate the specificity of TRABID more quantitatively, we developed a quantitative deubiquitinase assay. Previous work has generally followed the cleavage of a fixed concentration of diubiquitin at varying enzyme concentrations³⁷ or the cleavage of high concentrations of ubiquitin chains as a function of time³⁸. These experiments provide a qualitative measure of specificity but are not compatible with extracting quantitative kinetic parameters. A method for extracting kinetic parameters for diubiquitin has been reported, but it requires ¹²⁵I labeling of the ubiquitin chains^{39,40}. To characterize the specificity of TRABID, after the cleavage of Lys63- and Lys29-linked diubiquitin we performed quantitative western blotting (**Fig. 12** and **Supplementary** **Fig. 7**) at substrate concentrations below the *K*ₘ of TRABID for each substrate. (We determined the *k*_{cat} and *K*ₘ of TRABID cleaving of Lys63-linked diubiquitin independently by the method of initial rates, using the change in anisotropy of a fluorescently labeled Lys63-linked diubiquitin. The *K*ₘ for TRABID cleaving of Lys29-linked diubiquitin comes from competition experiments. See **Supplementary Methods** and **Supplementary** **Fig. 8****.**) This approach allowed us to determine the specificity constant (*k*_{cat}/Kₘ) for TRABID cleaving of Lys63-linked diubiquitin to be 2.5 (±0.4)×10³ M⁻¹ s⁻¹ (which is comparable to that obtained independently by fluorescence anisotropy, 1.7 (±2.0)×10³ M⁻¹ s⁻¹), as well as a specificity constant for TRABID cleaving of Lys29-linked diubiquitin, 1.0 (±0.2)×10⁵ M⁻¹ s⁻¹. These data demonstrate that Lys29-linked diubiquitin is a 40-fold better substrate for TRABID than Lys63-linked diubiquitin and suggest that Lys29-linked ubiquitin may be a preferred substrate of TRABID *in vivo.* Given that OTU domain-containing proteins other than TRABID appear to be intrinsically specific for a subset of linkages, it is possible that other family members can cleave other atypical ubiquitin chains specifically, including Lys6-linked chains. Extensions of our approach should allow us to discover these activities.

### Discussion of Example 6

We have demonstrated that GOPAL, a powerful combination of genetic code expansion and chemoselective chemical reactions, can be used to synthesize proteins linked by specific isopeptide bonds. We have used this method to synthesize Lys6- and Lys29-linked diubiquitin, allowing structural characterization of Lys6-linked diubiquitin and profiling of deubiquitinases for Lys6- and Lys29-linked ubiquitin cleavage specificity for the first time.

Taken together, the deubiquitinase assays demonstrate that Lys6- and Lys29-linked diubiquitin are recognized and hydrolyzed efficiently by USP family deubiquitinases. The data extend the characterization of deubiquitinase specificity, demonstrating that deubiquitinases that are specific for Lys48 over the other previously synthesizable linkages (for example, OTUB1, A20) maintain this specificity with respect to Lys6- and Lys29-linked diubiquitin. However, we now reveal that TRABID is 40-fold more active toward Lys29-linked ubiquitin than Lys63-linked ubiquitin, whereas it is inactive against Lys6 linkages. This provides an impetus to study the role of Lys29 linkages in biological pathways such as Wnt signaling¹⁸. It will be interesting to analyze whether TRABID has Lys29-modified substrates. In addition to its OTU domain, TRABID also contains three N-terminal Npl4-type ZnF (NZF) domains that bind to Lys63-linked and linear ubiquitin chains⁷. It is possible that the NZF domains also bind to Lys29-linked chains or regulate the TRABID interaction with Lys63 linkages *in vivo.* Overall, the observations from the deubiquitinase profiling support the view that deubiquitinases are intrinsically highly specific. Lys6- and Lys29-linked diubiquitin will be important tools in identifying further deubiquitinases for these linkages.

Access to Lys6- and Lys29-linked ubiquitin chains may allow us to generate antibodies against these new linkages^{9,10} to further understand their cellular roles. Specific ubiquitin-binding domains (UBDs) that discriminate between different ubiquitin chains have been described⁸, and the ability to synthesize Lys6 and Lys29 linkages should accelerate the discovery of UBDs that may specifically recognize these linkages.

The crystal structure of Lys6-linked diubiquitin reveals a new compact, asymmetric conformation in which the proximal and distal ubiquitin moieties interact through distinct residues (**Fig. 10**). This conformation is different from the compact, symmetric conformation of Lys48-linked ubiquitin, in which the Ile44 patches of linked ubiquitin molecules interact with each other⁴¹ (**Fig. 10f**), and from the compact, asymmetric conformation of Lys11-linked diubiquitin, which does not involve the Ile44 region¹¹. The crystal structure of Lys6-linked diubiquitin is also distinct from that of Lys63-linked or linear ubiquitin chains, which adopt open conformations with no interactions between individual ubiquitin molecules⁷ (**Fig. 10f**). However, the crystal structure provides a static picture of the dynamic diubiquitin molecules, and Lys6 linkages, like other linkages⁴², may adopt additional conformations in solution. That said, a distinct structure for Lys6-linked ubiquitin chains may provide an explanation for the proposed distinct biological function of this linkage type in the cell^{43,44}.

GOPAL may be applied to the synthesis of other isopeptide-linked proteins, including SUMOylated and Neddylated protein targets, though the method in its current form does require that the protein can be reversibly refolded. More broadly, the strategy for differentiating chemically identical residues in a protein by the site-specific encoding of a protected amino acid, chemical protection of other occurrences of the amino acid and deprotection of the encoded protected amino acid will allow the range of residue-selective and chemoselective reactions that have developed over many years of protein and peptide chemistry⁴⁵ to be applied for site-selective protein modification. As the method uses genetic encoding to define the site of modification or ligation, it is applicable to modifications at any site in a protein.

### METHODS

**Global protection of UbBocLys6 and UbBocLys29 with Cbz-Osu**. Lyophilized UbBocLys6 (20 mg, 2.3 µmol) was dissolved in DMSO (1.7 ml), and DIEA (67 µl) was added. While the mixture was stirred, Cbz-OSu (4.81 mg, 19.3 µmol) was added to the reaction. After 2 h of stirring at 25 °C, the reaction was transferred into cold ether (17 ml) and briefly vortexed. The precipitate was collected by centrifugation, and the ether layer was discarded. The pellet was washed with ice-cold ether (17 ml) and then air dried. For UbBocLys29, this and subsequent procedures were repeated pro rata to account for the reduced expression yield of UbBocLys29-His₆.

**Removal of Boc protecting group.** The dry, globally protected peptide UbBocLys6(Cbz₇₋₈), obtained from 10 mg of UbBocLys6, was dissolved in cold 9:6 TFA/H₂O (1.28 ml) and incubated at 4 °C for 90 min. The selectively deprotected peptide was then precipitated and washed with ice-cold ether (2 × 13 ml). The aqueous and ether layers were removed, and the peptide was left to air dry.

**Global protection of Ub-MES thioester with Cbz-Osu.** Lyophilized UbSR (10 mg, 1.15 µmol) was dissolved in DMSO (833 µl), and DIEA (17 µl) was added. While the mixture was stirred, Cbz-OSu (2.75 mg, 11.5 µmol) was added to the reaction. The reaction was stirred for 2 h at 25 °C and then transferred into cold ether (8.5 ml) and briefly vortexed. The precipitate was collected by centrifugation, and the ether layer was discarded. The pellet was washed with ice-cold ether (8.5 ml) and air dried.

**Specific isopeptide bond formation.** UbLys6(Cbz₇₋₈) (2.1 mg, 220 nmol) and UbSR(Cbz₇₋₈) (3.3 mg, 336 nmol) were dissolved in DMSO (90 µl). DIEA (4 µl), H-OSu (0.39 mg, 3.36 µmol) and AgNO₃ (57 µg, 336 nmol) were added. The reaction was incubated in the dark at 25 °C for 16 h. The crude mixture was precipitated with cold ether (1 ml), washed with cold ether (1 ml) and air dried. The proteins were dissolved in an ice-cold cocktail (5 mg ml⁻¹) consisting of 55% TFA, 35% DMS and 10%TFMSA. After being stirred at 0 °C for 90 min, the proteins were precipitated with ten volumes of cold ether and 0.5% (v/v) pyridine. A heavy precipitate formed, which was washed with cold ether, collected and dried. The dried precipitate was dissolved in buffer (100 mM Na₂HPO₄, pH 7.4; 8 M urea; 500 mM NaCl) at a protein concentration of approximately 0.5 mg ml⁻¹ and dialyzed overnight against the same buffer (1 liter) using a 3-kDa MWCO membrane (Spectrum Labs). The sample was then transferred to a fresh dialysis membrane and dialyzed overnight against folding buffer (20 mM Na₂HPO₄, pH 7.4; 100 mM NaCl). The protein was buffer exchanged into IEX buffer A (ammonium acetate, pH 4.5) using an Amicon Ultra-15 3-kDa MWCO centrifugal filter device (Millipore). The sample was filtered (0.45 µM) and loaded onto a pre-equilibrated MonoS 5/50 GL column (GE Life Sciences) at a flow rate of 0.5 ml min⁻¹ using an ÄKTA FPLC system. The flow was increased to 2 ml min⁻¹ and a gradient running to 60% IEX buffer B (ammonium acetate pH 4.5, 1 M NaCl) over 10 min was applied. Fractions (0.5 ml) were collected, and those containing Lys6-linked diubiquitin were determined by SDS-PAGE. The fractions were pooled and exchanged into IEX buffer A again using a centrifugal filter device. The sample was reapplied to the equilibrated MonoS column, and at a flow rate of 2 ml min⁻¹, a gradient running to 60% buffer B over 45 min was applied. Fractions were collected in 1-ml volumes, and those containing pure Lys6-linked- diubiquitin were pooled and concentrated to 1 mg ml⁻¹ (200-300 µg, 5-8% yield). Lys6-linked diubiquitin was then dialyzed overnight against storage buffer (10 mM Tris-HCl, pH 7.6) using a Dispo Biodialyzer 5-kDa MWCO (The Nest Group Inc.). The Lys6-linked diubiquitin samples were frozen at -20 °C for storage. Preparation of Lys29-linked diubiquitin was carried out as described for Lys6-linked diubiquitin, except UbLys29(Cbz₇₋₈) was used in the isopeptide bond-forming reaction.

**Other methods.** Detailed methods for the cloning of acceptor ubiquitin (UbTAG6-His₆ and UbTAG29-His₆), the cloning of donor ubiquitin Ub1-76-thioester, the preparation of donor ubiquitin Ub1-76,-thioester, deubiquitinase generation and Lys63 dimer generation, and preparation of acceptor ubiquitin (UbBocLys6 and UbBocLys29) can be found in the **Supplementary Methods,** as can methods for qualitative and quantitative deubiquitinase assays using silver staining and quantitative western blot and the analytical procedures for analyzing kinetic data.. Methods for (UbLys6)₂ crystallization, structure determination and refinement, and protein mass spectrometry can likewise be found in the **Supplementary Methods.**

**Accession codes.** The coordinates of Lys6-linked diubiquitin have been deposited with the Protein Data Bank, accession code 2xk5.

### References for example 6.

1. Chen, Z.J. & Sun, L.J. Nonproteolytic functions of ubiquitin in cell signaling. Mol. Cell 33, 275-286 (2009).
2. Komander, D. The emerging complexity of protein ubiquitination. Biochem. Soc. Trans. 37, 937-953 (2009).
3. Ikeda, F. & Dikic, I. Atypical ubiquitin chains: new molecular signals. 'Protein modifications: beyond the usual suspects' review series. EMBO Rep. 9, 536-542 (2008).
4. Peng, J. et al. A proteomics approach to understanding protein ubiquitination. Nat. Biotechnol. 21, 921-926 (2003).
5. Xu, P. et al. Quantitative proteomics reveals the function of unconventional ubiquitin chains in proteasomal degradation. Cell 137, 133-145 (2009).
6. Hershko, A. & Ciechanover, A. The ubiquitin system. Annu. Rev. Biochem. 67, 425-479 (1998).
7. Komander, D. et al. Molecular discrimination of structurally equivalent Lys 63-linked and linear polyubiquitin chains. EMBO Rep. 10, 466-473 (2009).
8. Dikic, I., Wakatsuki, S. & Walters, K.J. Ubiquitin-binding domains - from structures to functions. Nat. Rev. Mol. Cell Biol. 10, 659-671 (2009).
9. Wang, H. et al. Analysis of nondegradative protein ubiquitylation with a monoclonal antibody specific for lysine-63-linked polyubiquitin. Proc. Natl. Acad. Sci. USA 105, 20197-20202 (2008).
10. Newton, K. et al. Ubiquitin chain editing revealed by polyubiquitin linkage-specific antibodies. Cell 134, 668-678 (2008).
11. Bremm, A., Freund, S.M.V. & Komander, D. Lys11-linked ubiquitin chains adopt compact conformations and are preferentially hydrolysed by the deubiquitinase Cezanne. Nat. Struct. Mol. Biol. 17, 939-947 (2010)
12. Chatterjee, C., McGinty, R.K., Pellois, J.-P. & Muir, T.W. Auxiliary-mediated site-specific peptide ubiquitylation. Angew. Chem. Int. Edn Engl. 46, 2814-2818 (2007).
13. McGinty, R.K., Kim, J., Chatterjee, C., Roeder, R. & Muir, T. Chemically ubiquitylated histone H2B stimulates hDot1L-mediated intranucleosomal methylation. Nature 453, 812-816 (2008).
14. Yang, R., Pasunooti, K., Li, F., Liu, X. & Liu, C. Dual native chemical ligation at lysine. J. Am. Chem. Soc. 131, 13592-13593 (2009).
15. Ajish Kumar, K.S., Haj-Yahya, M., Olschewski, D., Lashuel, H.A. & Brik, A. Highly efficient and chemoselective peptide ubiquitylation. Angew. Chem. Int. Edn Engl. 48, 8090-8094 (2009).
16. Li, X., Fekner, T., Ottesen, J.J. & Chan, M.K. A pyrrolysine analogue for site-specific protein ubiquitination. Angew. Chem. Int. Edn Engl. 48, 9184-9187 (2009).
17. Hodgins, R.R., Ellison, K.S. & Ellison, M.J. Expression of a ubiquitin derivative that conjugates to protein irreversibly produces phenotypes consistent with a ubiquitin deficiency. J. Biol. Chem. 267, 8807-8812 (1992).
18. Tran, H., Hamada, F., Schwarz-Romond, T. & Bienz, M. Trabid, a new positive regulator of Wnt-induced transcription with preference for binding and cleaving K63-linked ubiquitin chains. Genes Dev. 22, 528-542 (2008).
19. Srinivasan, G., James, C.M. & Krzycki, J.A. Pyrrolysine encoded by UAG in Archaea: charging of a UAG-decoding specialized tRNA. Science 296, 1459-1462 (2002).
20. Ambrogelly, A. et al. Pyrrolysine is not hardwired for cotranslational insertion at UAG codons. Proc. Natl. Acad. Sci. USA 104, 3141-3146 (2007).
21. Neumann, H., Peak-Chew, S.Y. & Chin, J. W. Genetically encoding N(epsilon)-acetyllysine in recombinant proteins. Nat. Chem. Biol. 4, 232-234 (2008).
22. Polycarpo, C.R. et al. Pyrrolysine analogues as substrates for pyrrolysyl-tRNA synthetase. FEBS Lett. 580, 6695-6700 (2006).
23. Kawakami, T. et al. Polypeptide synthesis using an expressed peptide as a building block for condensation with a peptide thioester: application to the synthesis of phosphorylated p21Max protein(1-101). J. Pept. Sci. 7, 474-487 (2001).
24. Aimoto, S. Polypeptide synthesis by the thioester method. Biopolymers 51, 247-26S (1999).
25. Tan, Z., Shang, S., Halkina, T., Yuan, Y. & Danishefsky, S.J. Toward homogeneous erythropoietin: non-NCL-based chemical synthesis of the Gln(78)-Arg(l66) glycopeptide domain. J. Am. Chem. Soc. 131, 5424-5431 (2009).
26. Tam, J.P., Heath, W.F. & Merrifield, R.B. Mechanisms for the removal of benzyl protecting groups in synthetic peptides by trifluoromethanesulfonic acid trifluoroacetic-acid dimethyl sulfide. J. Am. Chem. Soc. 8, 5242-5251 (1986).
27. Pickart, C.M. & Raasi, S. Controlled synthesis of polyubiquitin Chains. Methods Enzymol. 399, 21-36 (2005).
28. Komander, D., Clague, M.J. & Urbe, S. Breaking the chains: structure and function of the deubiquitinases. Nat. Rep. Mol. Cell Biol. 10, 550-563 (2009).
29. Fushman, D. & Walker, O. Exploring linkage dependence of polyubiquitin conformations using molecular modeling. J. Mol. Biol. 395, 803-814 (2010).
30. Wang, T. et al. Evidence for bidentate substrate binding as the basis for the K48 linkage specificity of otubain 1. J. Mol. Biol. 386, 1011-1023 (2009).
31. Cooper, E.M. et al. K63-specific deubiquitination by two JAMM/MPN+ complexes: BRISC-associated Brcc36 and proteasomal Pohl. EMBO J. 28, 621-631 (2009).
32. Popp, M.W., Artavanis-Tsakonas, K. & Ploegh, H.L. Substrate filtering by the active site crossover loop in UCHL3 revealed by sortagging and gain-of-function mutations. J. Biol. Chem. 284, 3593-3602 (2009).
33. Reyes-Turcu, F.E. et al. The ubiquitin binding domain ZnF UBP recognizes the C-terminal diglycine motif of unanchored ubiquitin. Cell 124, 1197-1208 (2006).
34. Komander, D. et al. The structure of the CYLD USP domain explains its specificity for Lys63-linked polyubiquitin and reveals a B box module. Mol. Cell 29, 451-464 (2008).
35. McCullough, J., Clague, M.J. & Urbe, S. AMSH is an endosome-associated ubiquitin isopeptidase. J. Cell Biol. 166, 487-492 (2004).
36. Sato, Y. et al. Structural basis for specific cleavage of Lys 63-linked polyubiquitin chains. Nature 455, 358-362 (2008).
37. Winborn, B.J. et al. The deubiquitinating enzyme ataxin-3, a polyglutamine disease protein, edits Lys63 linkages in mixed linkage ubiquitin chains. J. Biol. Chem. 283, 26436-26443 (2008).
38. Komander, D. et al. Molecular discrimination of structurally equivalent Lys 63-linked and linear polyubiquitin chains. EMBO Rep. 10, 466-473 (2009).
39. Wang, T. et al. Evidence for bidentate substrate binding as the basis for the K48 linkage specificity of otubain 1. J. Mol. Biol. 386, 1011-1023 (2009).
40. Cooper, E.M., Boeke, J.D. & Cohen, R.E. Specificity of the BRISC deubiquitinating enzyme is not due to selective binding to Lys63-linked polyubiquitin. J. Biol. Chem. 285, 10344-10352 (2010).
41. Cook, W.J., Jeffrey, L.C., Carson, M., Chen, Z. & Pickart, C.M. Structure of a diubiquitin conjugate and a model for interaction with ubiquitin conjugating enzyme (E2). J. Biol. Chem. 267, 16467-16471 (1992).
42. Eddins, M.J., Varadan, R., Fushman, D., Pickart, C.M. & Wolberger, C. Crystal structure and solution NMR studies of Lys48-linked tetraubiquitin at neutral pH. J. Mol. Biol. 367, 204-211 (2007).
43. Wu-Baer, F., Lagrazon, K., Yuan, W. & Baer, R. The BRCA1/BARD1 heterodimer assembles polyubiquitin chains through an unconventional linkage involving lysine residue K6 of ubiquitin. J. Biol. Chem. 278, 34743-34746 (2003).
44. Nishikawa, H. et al. BRCA1-associated protein 1 interferes with BRCA1BARD1 RING heterodimer activity. Cancer Res. 69, 111-119 (2009).
45. Hermanson, G.T. Bioconjugate Techniques 2nd ed. (Academic Press, 2008).

### References other than for example 6.

1 Chen, Z.J. & Sun, L.J., Nonproteolytic functions of ubiquitin in cell signaling. Mol Cell 33 (3), 275-286 (2009).
2 Komander, D., The emerging complexity of protein ubiquitination. Biochem Soc Trans 37 (Pt 5), 937-953 (2009).
3 Ikeda, F. & Dikic, I., Atypical ubiquitin chains: new molecular signals. 'Protein Modifications: Beyond the Usual Suspects' review series. EMBO Rep 9 (6), 536-542 (2008).
4 Peng, J. et al., A proteomics approach to understanding protein ubiquitination. Nat Biotechnol 21 (8), 921-926 (2003).
5 Xu, P. et al., Quantitative proteomics reveals the function of unconventional ubiquitin chains in proteasomal degradation. Cell 137 (1), 133-145 (2009).
6 Hershko, A. & Ciechanover, A., The ubiquitin system. Annu Rev Biochem 67, 425-479 (1998).
7 Komander, D. et al., Molecular discrimination of structurally equivalent Lys 63-linked and linear polyubiquitin chains. EMBO Rep 10 (5), 466-473 (2009).
8 Dikic, I., Wakatsuki, S., & Walters, K.J., Ubiquitin-binding domains - from structures to functions. Nat Rev Mol Cell Biol 10 (10), 659-671 (2009).
9 Wang, H. et al., Analysis of nondegradative protein ubiquitylation with a monoclonal antibody specific for lysine-63-linked polyubiquitin. Proc Natl Acad Sci U S A 105 (51), 20197-20202 (2008).
10 Newton, K. et al., Ubiquitin chain editing revealed by polyubiquitin linkage-specific antibodies. Cell 134 (4), 668-678 (2008).
11 Chatterjee, C., McGinty, R.K., Pellois, J.-P., & Muir, T.W., Auxiliary-mediated site-specific peptide ubiquitylation. Angew Chem Int Ed Engl 46 (16), 2814-2818 (2007).
12 McGinty, R., Kim, J., Chatterjee, C., Roeder, R., & Muir, T., Chemically ubiquitylated histone H2B stimulates hDot1L-mediated intranucleosomal methylation. Nature (2008).
13 Yang, R., Pasunooti, K., Li, F., Liu, X., & Liu, C., Dual Native Chemical Ligation at Lysine. Journal of the American Chemical Society (2009).
14 Ajish Kumar, K.S., Haj-Yahya, M., Olschewski, D., Lashuel, H.A., & Brik, A., Highly efficient and chemoselective peptide ubiquitylation. Angew Chem Int Ed Engl 48 (43), 8090-8094 (2009).
15 Li, X., Fekner, T., Ottesen, J.J., & Chan, M.K., A pyrrolysine analogue for site-specific protein ubiquitination. Angew Chem Int Ed Engl 48 (48), 9184-9187 (2009).
16 Hodgins, R.R., Ellison,, K.S., & Ellison, M.J., Expression of a ubiquitin derivative that conjugates to protein irreversibly produces phenotypes consistent with a ubiquitin deficiency. J Biol Chem 267 (13), 8807-8812 (1992).
17 Tran, H., Hamada, F., Schwarz-Romond, T., & Bienz, M., Trabid, a new positive regulator of Wnt-induced transcription with preference for binding and cleaving K63-linked ubiquitin chains. Genes Dev 22 (4), 528-542 (2008).
18 Srinivasan, G., James, C.M., & Krzycki, J.A., Pyrrolysine encoded by UAG in Archaea: charging of a UAG-decoding specialized tRNA. Science 296 (5572), 1459-1462 (2002).
19 Ambrogelly, A. et al., Pyrrolysine is not hardwired for cotranslational insertion at UAG codons. Proc Natl Acad Sci U S A 104 (9), 3141-3146 (2007).
20 Neumann, H., Peak-Chew, S.Y., & Chin, J.W., Genetically encoding N(epsilon)-acetyllysine in recombinant proteins. Not Chem Biol 4 (4), 232-234 (2008).
21 Polycarpo, C.R. et al., Pyrrolysine analogues as substrates for pyrrolysyl-tRNA synthetase. FEBS Lett 580 (28-29), 6695-6700 (2006).
22 Kawakami, T. et al., Polypeptide synthesis using an expressed peptide as a building block for condensation with a peptide thioester: application to the synthesis of phosphorylated p21 Max protein(1-101). J Pept Sci 7 (9), 474-487 (2001).
23 Aimoto, S., Polypeptide synthesis by the thioester method. Biopolymers 51 (4), 247-265 (1999).
24 Tan, Z., Shang, S., Halkina, T., Yuan, Y., & Danishefsky, S.J., Toward Homogeneous Erythropoietin: Non-NCL-Based Chemical Synthesis of the Gln(78)-Arg(166) Glycopeptide Domain. J Am Chem Soc 131 (15), 5424-5431 (2009).
25 Tam, J.P., Heath, W.F., & Merrifield, R.B., Mechanisms for the removal of benzyl protecting groups in synthetic peptides by trifluoromethanesulfonic acid trifluoroacetic-acid dimethyl sulfide. Journal of the American Chemical Society 108 (17)', 5242-5251 (1986).
26 Pickart, C.M. & Raasi, S., Controlled Synthesis of Polyubiquitin Chains. Methods in Enzymology 399, 21-36 (2005).
27 Komander, D., Clague, M.J., & Urbe, S., Breaking the chains: structure and function of the deubiquitinases. Nat Rev Mol Cell Biol 10 (8), 550-563 (2009).
28 Emsley, P. & Cowtan, K., Coot: model-building tools for molecular graphics. Acta Crystallogr D Biol Crystallogr 60 (Pt 12 Pt 1), 2126-2132 (2004).
29 Wang, T. et al., Evidence for bidentate substrate binding as the basis for the K48 linkage specificity of otubain 1. J Mol Biol 386 (4), 1011-1023 (2009).
30 Cooper, E.M. et al., K63-specific deubiquitination by two JAMM/MPN+ complexes: BRISC-associated Brcc36 and proteasomal Pohl. EMBO J (2009).
31 Popp, M.W., Artavanis-Tsakonas, K., & Ploegh, H.L., Substrate Filtering by the Active Site Crossover Loop in UCHL3 Revealed by Sortagging and Gain-of-function Mutations. J Biol Chem 284 (6), 3593-3602 (2009).
32 Komander, D. et al., The structure of the CYLD USP domain explains its specificity for Lys63-linked polyubiquitin and reveals a B box module. Mol Cell 29 (4), 451-464 (2008).
33 Reyes-Turcu, F.E. et al., The ubiquitin binding domain ZnF UBP recognizes the C-terminal diglycine motif of unanchored ubiquitin. Cell 124 (6), 1197-1208 (2006).
34 McCullough, J., Clague, M.J., & Urbe, S., AMSH is an endosome-associated ubiquitin isopeptidase. J Cell Biol 166 (4), 487-492 (2004).
35 Sato, Y. et al., Structural basis for specific cleavage of Lys 63-linked polyubiquitin chains. Nature 455 (7211), 358-362 (2008).
36 Wu-Baer, F., Lagrazon, K., Yuan, W., & Baer, R., The BRCA1/BARD1 heterodimer assembles polyubiquitin chains through an unconventional linkage involving lysine residue K6 of ubiquitin. J Biol Chem 278 (37), 34743-34746 (2003).
37 Nishikawa, H. et al., BRCA1-associated protein 1 interferes with BRCA1/BARD1 RING heterodimer activity. Cancer Res 69 (1), 111-119 (2009).
38 Venkitaraman, A.R., Cancer susceptibility and the functions of BRCA1 and BRCA2. Cell 108 (2), 171-182 (2002).
39 Al-Hakim, A.K. et al., Control of AMPK-related kinases by USP9X and atypical Lys(29)/Lys(33)-linked polyubiquitin chains. Biochem J 411 (2), 249-260 (2008).
40 Chastagner, P., Israel, A., & Brou, C., Itch/AlP4 mediates Deltex degradation through the formation of K29-linked polyubiquitin chains. EMBO Rep 7 (11), 1147-1153 (2006).
41 Hermanson, G.T., Bioconjugate Techniques, 2nd ed. (Academic Press, 2008).
42 Neumann, H. et al., A method for genetically installing site-specific acetylation in recombinant histones defines the effects of H3 K56 acetylation. Mol Cell 36 (1). 153-163 (2009).
43 Larsen, C.N., Price, J.S., .& Wilkinson, K.D., Substrate binding and catalysis by ubiquitin C-terminal hydrolases: identification of two active site residues. Biochemistry 35 (21), 6735-6744 (1996).
44 Edelmann, M.J. et al., Structural basis and specificity of human otubain 1-mediated deubiquitination. Biochem J 418 (2), 379-390 (2009).
45 Adams, P.D. et al., PHENIX: building new software for automated crystallographic structure determination. Acta Crystallogr D Biol Crystallogr 58 (Pt 11), 1948-1954 (2002).
46 Cook, W.J., Jeffrey, L.C., Carson, M., Chen, Z., & Pickart, C.M., Structure of a diubiquitin conjugate and a model for interaction with ubiquitin conjugating enzyme (E2). J Biol Chem 267 (23), 16467-16471 (1992).

## Claims

1. A method of modifying a specific lysine residue in a polypeptide comprising at least two lysine residues, said method comprising
(a) providing a polypeptide comprising a target lysine residue, protected by a first protecting group, and at least one further lysine residue;
wherein providing the polypeptide comprises
(i) providing a nucleic acid encoding the polypeptide which nucleic acid comprises an orthogonal codon encoding the target lysine;
(ii) translating said nucleic acid in the presence of an orthogonal tRNA. synthetase/tRNA pair capable of recognising said orthogonal codon and incorporating said target lysine residue protected by a first protecting group into the polypeptide chain;
(b) treating the polypeptide to protect said further lysine residue(s), wherein the protecting group for said further lysine residues is different to the protecting group for the target lysine residue;
(c) selectively deprotecting the target lysine residue; and
(d) modifying the deprotected lysine residue of (c).

2. A method according to claim 1 wherein said orthogonal codon comprises TAG, said tRNA comprises *Mb*tRNA_{CUA} and said tRNA synthetase comprises *Mb*PylRS.

3. A method according to any preceding claim wherein step (b) comprises treating the polypeptide with *N-*(benzyloxycarbonyloxy)succinimide (Cbz-OSu) in basic DMSO.

4. A method according to any preceding claim wherein step (c) comprises treating the polypeptide with trifluoroacetic (TFA) acid in water.

5. A method according to any preceding claim wherein the modification of step (d) comprises
(i) activating thioester by conversion to *N*-hydroxysuccinimidyl ester in the presence of Ag(I);
(ii) adding a polypeptide to be joined to the target lysine; and
(iii) incubating to allow formation of a specific isopeptide bond.

6. A method according to any preceding claim wherein the modification of step (d) is carried out on the ε-amino group of the target lysine residue.

7. A method according to any preceding claim further comprising the step (e) deprotecting said further lysine residue(s):

8. A method according to claim 7 wherein step (e) comprises treating the polypeptide with a mixture of trifluoromethanesulfonic acid (TFMSA):trifluoroacetic acid (TFA):dimethylsulfide (DMS) in the ratio 1:3:6.

9. A method according to any preceding claim, wherein the modification of step (d) is the covalent linkage of a further polypeptide chain to the target lysine.

10. A method according to claim 9 wherein the further polypeptide chain is ubiquitin.

11. The method according to any of claims 1 to 6 wherein multiple modifications are made to the target lysine in step (d), and wherein steps (c)-(d) are repeated to produce a chain of polypeptides joined by covalent linkages through lysine residues.

12. A method according to any of claims 1 to 11, wherein the target lysine residue protected by a first protecting group is Nε-(t-butyloxycarbonyl)-L-lysine.

13. A method according to any of claims 1 to 11, wherein the protecting group for said further lysine residues is N-(benzyloxycarbonyloxy) succinimide (Cbz-Osu).

14. A method according to any of claims 1 to 11, wherein the polypeptide is ubiquitin.

## Patentansprüche

1. Verfahren zum Modifizieren eines spezifischen Lysinrests bei einem Polypeptid, das mindestens zwei Lysinreste umfasst, wobei das Verfahren Folgendes umfasst:
(a) Bereitstellen eines Polypeptids, das einen Ziel-Lysinrest, der durch eine erste Schutzgruppe geschützt ist sowie mindestens einen weiteren Lysinrest umfasst;
wobei das Bereitstellen des Polypeptids Folgendes umfasst:
(i) Bereitstellen einer für das Polypeptid kodierenden Nukleinsäure, wobei die Nukleinsäure ein orthogonales Kodon umfasst, das für das Ziel-Lysin kodiert;
(ii) Translatieren der Nukleinsäure in Gegenwart eines orthogonalen tRNA-Synthetase/tRNA Paares, welches das orthogonale Kodon erkennen kann und Einbauen des Ziel-Lysinrests, der durch eine erste Schutzgruppe geschützt ist, in die Polypeptidkette;
(b) Behandeln des Polypeptids, um den/die weiteren Lysinrest(e) zu schützen, wobei sich die Schutzgruppe für die weiteren Lysinreste von der Schutzgruppe für den Ziel-Lysinrest unterscheidet;
(c) selektives Entschützen des Ziel-Lysinrests; und
(d) Modifizieren des entschützten Lysinrests aus (c).

2. Verfahren nach Anspruch 1, wobei das orthogonale Kodon TAG umfasst, die tRNA *Mb*tRNA_{CUA} umfasst und die tRNA Synthetase *Mb*PylRS umfasst.

3. Verfahren nach einem der vorherigen Ansprüche, wobei Schritt (b) das Behandeln des Polypeptids mit N-(Benzyloxycarbonyloxy)succinimid (Cbz-OSu) in basischem DMSO umfasst.

4. Verfahren nach einem der vorherigen Ansprüche, wobei Schritt (c) das Behandeln des Polypeptids mit Trifluoressigsäure (TFA) in Wasser umfasst.

5. Verfahren nach einem der vorherigen Ansprüche, wobei die Modifizierung von Schritt (d) Folgendes umfasst:
(i) Aktivieren von Thioester durch Umwandlung zu *N-*Hydroxysuccinimidylester in der Gegenwart von Ag(I);
(ii) Hinzufügen eines Polypeptids, das mit dem Ziel-Lysin verbunden werden soll; und
(iii) Inkubieren, um die Bildung einer spezifischen Isopeptidbindung zu ermöglichen.

6. Verfahren nach einem der vorherigen Ansprüche, wobei die Modifikation in Schritt (d) an der ε-Aminogruppe des Ziel-Lysinrests durchgeführt wird.

7. Verfahren nach einem der vorherigen Ansprüche, das ferner den Schritt (e) des Entschützens des/der weiteren Lysinrests/Lysinreste umfasst.

8. Verfahren nach Anspruch 7, wobei Schritt (e) das Behandeln des Polypeptids mit einem Gemisch von Trifluormethansulfonsäure (TFMSA):Trifluoressigsäure (TFA):Dimethylsulfid (DMS) im Verhältnis 1:3:6 umfasst.

9. Verfahren nach einem der vorherigen Ansprüche, wobei die Modifikation in Schritt (d) die kovalente Bindung einer weiteren Polypeptidkette an das Ziel-Lysin ist.

10. Verfahren nach Anspruch 9, wobei die weitere Polypeptidkette Ubiquitin ist.

11. Verfahren nach einem der Ansprüche 1 bis 6, wobei mehrere Modifikationen an dem Ziel-Lysin in Schritt (d) vorgenommen werden und wobei die Schritte (c) - (d) wiederholt werden, um eine Kette von Polypeptiden zu erzeugen, die durch kovalente Bindungen über Lysinreste verbunden sind.

12. Verfahren nach einem der Ansprüche 1 bis 11, wobei der durch eine erste Schutzgruppe geschützte Ziel-Lysinrest N_{ε}-(t-Butyloxycarbonyl)-L-lysin ist.

13. Verfahren nach einem der Ansprüche 1 bis 11, wobei die Schutzgruppe für die weiteren Lysinreste N-(Benzyloxycarbonyloxy)succinimid (Cbz-OSu) ist.

14. Verfahren nach einem der Ansprüche 1 bis 11, wobei das Polypeptid Ubiquitin ist.

## Revendications

1. Procédé de modification d'un résidu de lysine spécifique dans un polypeptide comprenant au moins deux résidus de lysine, ledit procédé comprenant les étapes consistant à :
(a) fournir un polypeptide comprenant un résidu de lysine cible protégé par un premier groupement protecteur, et au moins un autre résidu de lysine ;
dans lequel la fourniture du polypeptide comprend les étapes consistant à :
(i) fournir un acide nucléique codant pour le polypeptide, lequel acide nucléique comprend un codon orthogonal codant pour la lysine cible ;
(ii) traduire ledit acide nucléique en présence d'un tARN orthogonal, la paire synthétase/tARN étant capable de reconnaître ledit codon orthogonal, et incorporer ledit résidu de lysine cible protégé par un premier groupement protecteur dans la chaîne polypeptidique ;
(b) traiter le polypeptide pour protéger le ou lesdits autres résidus de lysine, dans lequel le groupement protecteur pour lesdits autres résidus de lysine est différent du groupement protecteur pour le résidu de lysine cible ;
(c) déprotéger sélectivement le résidu de lysine cible ; et
(d) modifier le résidu de lysine déprotégé de (c).

2. Procédé selon la revendication 1, dans lequel ledit codon orthogonal comprend le TAG, ledit tARN comprend le *Mb*tARN_{CUA} et ladite tARN synthétase comprend la *Mb*PylRS.

3. Procédé selon l'une quelconque des revendications précédentes, dans lequel l'étape (b) comprend le traitement du polypeptide avec du N-(benzyloxycarbonyloxy)succinimide (Cbz-OsU) dans du DMSO basique.

4. Procédé selon l'une quelconque des revendications précédentes, dans lequel l'étape (c) comprend le traitement du polypeptide avec de l'acide trifluoroacétique (TFA) dans de l'eau.

5. Procédé selon l'une quelconque des revendications précédentes, dans lequel la modification de l'étape (d) comprend :
(i) l'activation de thioester par conversion en ester de N-hydroxysuccinimidyle en présence d'Ag(I) ;
(ii) l'addition d'un polypeptide à joindre à la lysine cible ; et
(iii) l'incubation pour permettre la formation d'une liaison isopeptidique spécifique.

6. Procédé selon l'une quelconque des revendications précédentes, dans lequel la modification de l'étape (d) est effectuée sur le groupement ε-amino du résidu de lysine cible.

7. Procédé selon l'une quelconque des revendications précédentes, comprenant en outre l'étape (e) de déprotection du ou desdits autres résidus de lysine.

8. Procédé selon la revendication 7, dans lequel l'étape (e) comprend le traitement du polypeptide avec un mélange d'acide trifluorométhanesulfonique (TFMSA), d'acide trifluoroacétique (TFA) et de sulfure de diméthyle (DMS) dans le rapport de 1:3:6.

9. Procédé selon l'une quelconque des revendications précédentes, dans lequel la modification de l'étape (d) est la liaison covalente d'une autre chaîne polypeptidique avec la lysine cible.

10. Procédé selon la revendication 9, dans lequel l'autre chaîne polypeptidique est l'ubiquitine.

11. Procédé selon l'une quelconque des revendications 1 à 6, dans lequel de multiples modifications sont faites sur la lysine cible à l'étape (d) et dans lequel les étapes (c)-(d) sont répétées pour produire une chaîne de polypeptides joints par des liaisons covalentes via des résidus de lysine.

12. Procédé selon l'une quelconque des revendications 1 à 11, dans lequel le résidu de lysine cible protégé par un premier groupement protecteur est la N_{ε}-(t-butyloxycarbonyl)-L-lysine.

13. Procédé selon l'une quelconque des revendications 1 à 11, dans lequel le groupement protecteur pour lesdits autres résidus de lysine est le N-(benzyloxycarbonyloxy)succinimide (Cbz-Osu).

14. Procédé selon l'une quelconque des revendications 1 à 11, dans lequel le polypeptide est l'ubiquitine.


### SEQUENCE LISTING

<110> Medical Research Council
<120> Synthesis of specifically linked ubiquitin chains
<130> P3233GBOO
<160> 5
<170> PatentIn version 3.5
<210> 1
   <211> 419
   <212> PRT
   <213> Methanosarcina barkeri
<400> 1
   Met Asp Lys Lys Pro Leu Asp Val Leu Ile Ser Ala Thr Gly Leu Trp
   1               5                   10                  15
   Met Ser Arg Thr Gly Thr Leu His Lys Ile Lys His Tyr Glu Val Ser
               20                  25                  30
   Arg Ser Lys Ile Tyr Ile Glu Met Ala Cys Gly Asp His Leu Val Val
           35                  40                  45
   Asn Asn Ser Arg Ser Cys Arg Thr Ala Arg Ala Phe Arg His His Lys
       50                  55                  60
   Tyr Arg Lys Thr Cys Lys Arg Cys Arg Val Ser Asp Glu Asp Ile Asn
   65                  70                  75                  80
   Asn Phe Leu Thr Arg Ser Thr Glu Gly Lys Thr Ser Val Lys Val Lys
                   85                  90                  95
   Val Val Ser Ala Pro Lys Val Lys Lys Ala Met Pro Lys Ser Val Ser
               100                 105                 110
   Arg Ala Pro Lys Pro Leu Glu Asn Pro Val Ser Ala Lys Ala Ser Thr
           115                 120                 125
   Asp Thr Ser Arg Ser Val Pro Ser Pro Ala Lys Ser Thr Pro Asn Ser
       130                 135                 140
   Pro Val Pro Thr Ser Ala Pro Ala Pro Ser Leu Thr Arg Ser Gln Leu
   145                 150                 155                 160
   Asp Arg Val Glu Ala Leu Leu Ser Pro Glu Asp Lys Ile Ser Leu Asn
                   165                 170                 175
   Ile Ala Lys Pro Phe Arg Glu Leu Glu Ser Glu Leu Val Thr Arg Arg
               180                 185                 190
   Lys Asn Asp Phe Gln Arg Leu Tyr Thr Asn Asp Arg Glu Asp Tyr Leu
   195 200 205
   Gly Lys Leu Glu Arg Asp Ile Thr Lys Phe Phe Val Asp Arg Asp Phe
   210 215 220
   Leu Glu Ile Lys Ser Pro Ile Leu Ile Pro Ala Glu Tyr Val Glu Arg
   225 230 235 240
   Met Gly Ile Asn Asn Asp Thr Glu Leu Ser Lys Gln Ile Phe Arg Val
   245 250 255
   Asp Lys Asn Leu Cys Leu Arg Pro Met Leu Ala Pro Thr Leu Tyr Asn
   260 265 270
   Tyr Leu Arg Lys Leu Asp Arg Ile Leu Pro Asp Pro Ile Lys Ile Phe
   275 280 285
   Glu Val Gly Pro Cys Tyr Arg Lys Glu Ser Asp Gly Lys Glu His Leu
   290 295 300
   Glu Glu Phe Thr Met Val Asn Phe Cys Gln Met Gly Ser Gly Cys Thr
   305 310 315 320
   Arg Glu Asn Leu Glu Ser Leu Ile Lys Glu Phe Leu Asp Tyr Leu Glu
   325 330 335
   Ile Asp Phe Glu Ile Val Gly Asp Ser Cys Met Val Tyr Gly Asp Thr
   340 345 350
   Leu Asp Ile Met His Gly Asp Leu Glu Leu Ser Ser Ala Val Val Gly
   355 360 365
   Pro Val Pro Leu Asp Arg Glu Trp Gly Ile Asp Lys Pro Trp Ile Gly
   370 375 380
   Ala Gly Phe Gly Leu Glu Arg Leu Leu Lys Val Met His Gly Phe Lys
   385 390 395 400
   Asn Ile Lys Arg Ala Ser Arg Ser Glu Ser Tyr Tyr Asn Gly Ile Ser
   405 410 415
   Thr Asn Leu
<210> 2
   <211> 37
   <212> DNA
   <213> Artificial sequence
<220>
   <223> Forward primer for UBC ubiquitin gene
<400> 2
   cgcgcgccat ggagatcttc gtgaagaccc tgactgg 37
<210> 3
   <211> 60
   <212> DNA
   <213> Artificial Sequence
<220>
   <223> Reverse primer for UBC ubiquitin gene
<400> 3
   gccggatctc cgctcgagtt agtggtgatg atggtgatgc ccacctctga gacggaggac 60
<210> 4
   <211> 36
   <212> DNA
   <213> Artificial Sequence
<220>
   <223> Forward primer for ubiquitin gene
<400> 4
   ggtggtcata tgcagatctt cgtcaagacg ttaacc 36
<210> 5
   <211> 45
   <212> DNA
   <213> Artificial Sequence
<220>
   <223> Reverse primer for ubiquitin gene
<400> 5
   ggtggttgct cttccgcacc cgccacgcag tcttaagacc agatg 45